# EUROPÄISCHE PATENTANMELDUNG

(11) **EP 2 135 875 A1**
(43) Veröffentlichungstag der Anmeldung: **23.12.2009**
(21) Anmeldenummer: 09168889.5
(22) Anmeldetag: 22.10.2005
(51) Int. Cl.: C07K 7/06

(54) **Verfahren zur gezielten Herstellung von Lysobactinderivaten**

(30) Priorität: 05.11.2004 DE 102004053409
(62) Teilanmeldung aus: 05802611.3
(71) Anmelder: AiCuris GmbH & Co. KG, 42117 Wuppertal (DE)
(72) Erfinder: von Nussbaum, Franz, 40219 Düsseldorf (DE); Schröder, Werner, 42113 Wuppertal (DE); Fürstner, Chantal, 45478 Mülheim an der Ruhr (DE)
(74) Vertreter: Witte, Weller & Partner

(57) **Zusammenfassung**

Die Erfindung betrifft Verfahren zur gezielten Herstellung von Lysobactinderivaten durch kombinierte chemische und enzymatische Modifikationen. In besonderer Weise betrifft die Erfindung ein Verfahren zur Herstellung vom Lysobactinderivat 4-11 durch chemische Reduktion und Spaltung der entstehenden Produkte durch Chymotrypsin (Fig.1).

## Beschreibung

Die Erfindung betrifft Verfahren zur gezielten Herstellung von Lysobactinderivaten durch kombinierte chemische und enzymatische Modifikationen. In besonderer Weise betrifft die Erfindung ein Verfahren zur Herstellung vom Lysobactinfragment 4-11 durch chemische Reduktion und Spaltung der entstehenden Produkte durch Chymotrypsin.

Lysobactin ist ein cyclisches Depsipeptid, das aus einem Screeningprogramm zur Auffindung neuer in die Biosynthese bakterieller Zellwände eingreifender Antibiotika stammt (O'Sullivan J. et al. (1988) J. Antibiot. 41 (12), 1740-1744 und Bonner, D. P. et al. (1988) J. Antibiot. 41 (12), 1745-1751; Tymiak, A. A. et al. (1989) J. Org. Chem. 54, 1149-1157). Es zeigt eine hohe Wirksamkeit gegenüber Gram-positiven aeroben und anaeroben Bakterien. Ungewöhnlich ist die hohe Anzahl an nicht-proteinogenen Aminosäuren im Molekül. Neben den drei β-Hydroxyaminosäuren (2S,3R)-β-Hydroxy-Leucin, (2S,3R)-β-Hydroxy-Phenylalanin und (2S,3S)-β-Hydroxy-Asparagin kommen auch die D-Aminosäuren D-Leucin und D-Arginin sowie allo-Threonin vor. Diese Komplexität und die Größe des Naturstoffs Lysobactin stellen für gezielte chemische Modifikationen eine große Hürde dar.

Eine Aufgabe der vorliegenden Erfindung ist es daher, neue und alternative Syntheseverfahren zur gezielten Synthese von Lysobactinfragmenten zur Verfügung zu stellen, um die Herstellung von neuen Antibiotika unter Verwendung von Lysobactinfragmenten zu ermöglichen.

Einen Ausweg bietet die gezielte enzymatische Spaltung, die gezielte enzymatische Herstellung und die nachfolgende Verknüpfung von Lysobactinfragmenten in Kombination mit chemischen Modifikationsschritten, z. B. der Hydrierung..

Enzymatische Verdauexperimente von Lysobactin und der durch Hydrolyse erhaltenen offenkettigen Form ("open-chain Lysobactin"; Verbindung der Formel (I)) mit Enzymen wie Pepsin, Trypsin, Chymotrypsin und mucosalen Peptidase zeigten keinen (wie z. B. bei Pepsin) oder nur einen unzureichenden enzymatischen Verdau (R. A. Blackburn et. al. (1993) Drug Metab. Dispos. 21(4), 573-579). Zu einer sehr langsamen, ineffizienten enzymatischen Spaltung von Lysobactin kommt es erst nach Öffnung des Ringes durch Hydrolyse im verwendeten Puffer. Dies führt als unerwünschte Nebenreaktion zu einer Seitenketten-Deamidierung am (2S,3S)-β-Hydroxy-Asparagin. D.h. die β-Hydroxy-Asparagin-Einheit wandelt sich in eine β-Hydroxy-Aspartat-Einheit um.

Überraschender Weise wurde gefunden, dass das Lysobactinfragment 4-11 hocheffizient und quantitativ durch enzymatische Spaltung mit Chymotrypsin aus Dihydro-Lysobactin (Verbindung der Formel (II)) und Octahydro-Lysobactin (Verbindung der Formel (III)) sowie aus einem Gemisch beider Komponenten hergestellt werden kann. Die Spaltung geht so schnell vonstatten, dass praktisch nach dem Zusammengeben der Reaktionspartner (Substrat und Enzym) die Fragmente 1-3 und 4-11 entstehen. Unerwünschte Nebenreaktionen in den Aminosäure-Seitenketten finden nicht statt.

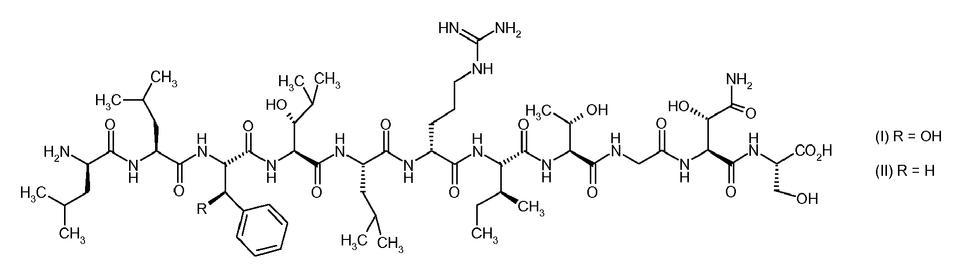

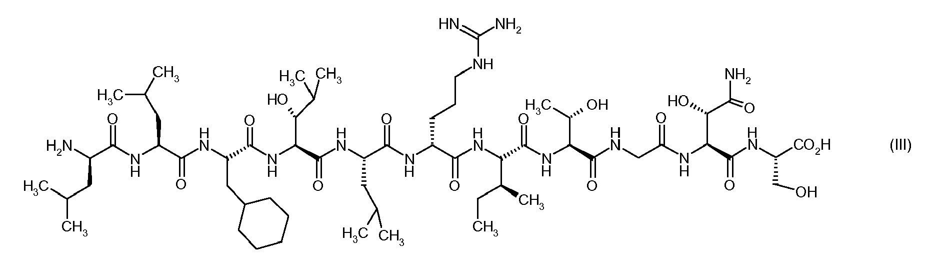

Dihydro-Lysobactin und Octahydro-Lysobactin erhält man durch hydrogenolytische Öffnung von Lysobactin mit Wasserstoff, wobei die (2S,3R)-β-Hydroxy-Phenylalanin-Einheit in eine Phenylalanin bzw. 3-Cyclohexylalanin-Einheit überführt wird. Die entstehenden Lysobactin-fragmente Dihydro-Lysobactin und Octahydro-Lysobactin werden dann für den enzymatischen Verdau eingesetzt.

Überraschenderweise sind Dihydro-Lysobactin und Octahydro-Lysobactin auch für andere Enzyme gute Substrate, so dass sich durch Auswahl des Enzyms auch andere Fragmente in hoher Ausbeute herstellen lassen.

Gegenstand der Erfindung ist ein Verfahren zur Herstellung von Dihydro-Lysobactin und/oder Octahydro-Lysobactin, bei dem Lysobactin durch hydrogenolytische Ringöffnung mit Wasserstoff in Gegenwart eines Hydrier-Katalysators in einem Lösungsmittel in Dihydro-Lysobactin und/oder Octahydro-Lysobactin überführt wird.

Hydrierkatalysatoren sind beispielsweise Palladium-, Ruthenium-, Rhodium-, Iridium- und PlatinKatalysatoren, oder Raney-Nickel. Diese Katalysatoren können als Salze (z. B. Platindioxid, Rhodium(III)chlorid) oder als Trägerkatalysatoren (z. B. Palladium auf Kohle (5-30%ig) oder Rhodium auf Kohle (5%ig)) eingesetzt werden. Geeignete Trägermaterialien für Trägerkatalysatoren sind beispielsweise Aktivkohle, Kieselgur, Kieselgel, Bentonite, Kaolin, Bimsstein, Alumosilicate oder Aluminiumoxid. Bevorzugtes Trägermaterial ist Aktivkohle.

Es können auch bimetallische Katalysatoren oder aber Mehrstoffkatalysatoren eingesetzt werden.

Bevorzugt sind Palladium-Katalysatoren, beispielsweise Palladium auf Kohle (5-30%ig), besonders bevorzugt ist Palladium auf Kohle (10%ig).

Die hydrogenolytische Ringöffnung erfolgt im Allgemeinen in einem Lösungsmittel, bevorzugt in einem Temperaturbereich von Raumtemperatur bis 150°C, bevorzugt in einem Temperaturbereich von Raumtemperatur bis 80°C, in einem Normaldruckbereich von Normaldruck bis 200 bar, bevorzugt in einem Druckbereich von 3 bis 80 bar.

Lösungsmittel sind beispielsweise Alkohole wie Methanol, Ethanol oder Isoropanol, oder Gemische der Alkohole mit Wasser, oder Essigsäure oder wässrige Lösungen von Essigsäure, oder THF-Wasser Gemische, oder Dioxan-Wasser-Gemische, oder aber ternäre Gemische der vorher genannten Lösungsmittel, z. B. Isopropanol-Wasser-Essigsäure. Bevorzugt ist ein Isopropanol-Wasser Gemisch.

Weiterer Gegenstand der Erfindung ist ein Verfahren zur Herstellung von Lysobactinfragment 4-11 und Lysobactinfragment 1-3, bei dem Dihydro-Lysobactin und/oder Octahydro-Lysobactin enzymatisch zum Lysobactinfragment 4-11 und Lysobactin-fragment 1-3 gespalten wird.

Bevorzugt ist eine enzymatische Spaltung von Dihydro-Lysobactin und/oder Octahydro-Lysobactin, wobei als Enzym eine eukaryontische Serinprotease oder eine mikrobielle Serinprotease verwendet wird.

Eukaryontische Serinproteasen sind beispielsweise Chymotrypsin, Cathepsin G, Chymase oder andere Enzyme der Chymotrypsinfamilie oder andere eukaryontische Serinproteasen, die nach aromatischen Aminosäuren spalten, bevorzugt ist Chymotrypsin.

Mikrobielle Serinproteasen sind beispielsweise Subtilisin, Proteinase K, Streptomyces protease A oder andere Enzyme die nach aromatischen Aminosäuren spalten, bevorzugt ist Subtilisin.

Die Erfindung umfasst weiterhin ein Verfahren zur enzymatischen Spaltung von Dihydro-Lysobactin und/oder Octahydro-Lysobactin zu kleineren Lysobactinfragmenten.

Weiterer Gegenstand der Erfindung ist dementsprechend ein Verfahren zur Herstellung von Lysobactinfragment 3-11 und/oder Lysobactinfragment 5-11 und/oder Lysobactinfragment 4-10 und/oder Lysobactinfragment 1-9, **dadurch gekennzeichnet, dass** Dihydro-Lysobactin und/oder Octahydro-Lysobactin enzymatisch zum Lysobactinfragment 3-11 und/oder Lysobactinfragment 5-11 und/oder Lysobactinfragment 4-10 und/oder Lysobactinfragment 1-9 gespalten wird.

Bevorzugt ist eine enzymatische Spaltung von Dihydro-Lysobactin und/oder Octahydro-Lysobactin, wobei als Enzym eine Metalloprotease oder eine Cysteinprotease verwendet wird.

Metalloproteasen sind beispielsweise Thermolysin oder Mycolysin.

Cysteinproteasen sind beispielsweise Papain, Bromelain oder Ficin.

Die enzymatische Spaltung erfolgt im Allgemeinen in einem wässrigen Spaltpuffer, unter Zugabe eines C₁-C₄-Alkohols oder Acetonitril, bevorzugt in einem Temperaturbereich von 10°C bis 40°C, bevorzugt in einem pH-Bereich von 6 bis 9 bei Normaldruck.

Ein wässriger Spaltpuffer enthält beispielsweise Ammoniumhydrogencarbonat und Harnstoff, oder Natriumphosphat, Cystein und EDTA, oder Natriumtetraborat, oder andere Zusatzstoffe mit denen ein Pufferbereich von pH 6 bis 9 abgedeckt wird, bevorzugt ist Ammoniumhydrogencarbonat und Harnstoff.

Der C₁-C₄-Alkohol ist beispielsweise Methanol, Ethanol oder Isopropanol, bevorzugt ist Methanol.

Besonders bevorzugt findet die enzymatische Spaltung in einem Temperaturbereich von 30°C bis 37°C statt.

Die Alkoholkonzentration im Reaktionsmedium beträgt 0% bis 40%, bevorzugt 10% bis 15%.

Das Verhältnis von Enzym zu Substrat (Dihydro-Lysobactin und/oder Octahydro-Lysobactin) beträgt 1:1 bis 1:4000, bevorzugt 1:25 bis 1:100.

Weiterer Gegenstand der Erfindung ist die Verwendung von Lysobactinfragment 4-11 zur Synthese von Lysobactin-Derivaten.

Bei diesen Lysobactin-Derivaten handelt es sich um Derivate, bei denen ein oder mehrere Aminosäuren im Ringsystem des Lysobactins ausgetauscht sind.

Weiterer Gegenstand der Erfindung ist ein Verfahren zur Herstellung von open-chain-Lysobactin-Derivaten, bei dem das Lysobactinfragment 4-11 mit einem Tripeptid mit einer C-terminalen aromatischen oder hydrophoben Aminosäure in einem Puffermedium unter Zugabe eines C₁₋₄-Alkohols umgesetzt wird, wobei das Tripeptid in Form der freien Säure oder eines Esters vorliegt und wobei die Konzentration des C₁₋₄-Alkohols im Reaktionsmedium größer als 40% ist.

Der C₁-C₄-Alkohol ist beispielsweise Methanol, Ethanol oder Isopropanol, bevorzugt ist Methanol.

Bevorzugt ist ein Verfahren zur enzymatischen Synthese von open-chain-Lysobactin-Derivaten aus dem Lysobactinfragment 4-11 und dem Tripeptid H-D-X-Y-Phe-OR oder H-D-X-Y-(3-cyclohexyl)Ala-OR in einem Puffermedium unter Zugabe von Methanol, wobei die Methanolkonzentration im Reaktionsmedium größer als 40% ist,
- R: für Wasserstoff oder C₁-C₄-Alkyl, bevorzugt für Ethyl oder Methyl, besonders bevorzugt für Methyl steht,
- D-X: für eine D-konfigurierte natürliche oder synthetische α-Aminosäure steht,
- und:
- Y: für eine L-konfigurierte natürliche oder synthetische α-Aminosäure steht.

Besonders bevorzugt ist ein Verfahren zur enzymatischen Synthese von open-chain-Lysobactin-Derivaten aus dem Lysobactinfragment 4-11 und dem Tripeptid H-D-Leu-Leu-Phe-OMethyl, H-D-Leu-Leu-Phe-OH, H-D-Leu-Leu-(3-cyclohexyl)Ala-OMethyl oder H-D-Leu-Leu-(3-cyclohexyl)Ala-OH, wobei als Enzym Chymotrypsin verwendet wird, in einem Puffermedium unter Zugabe von Methanol, wobei die Methanolkonzentration im Reaktionsmedium größer als 40% ist.

### Beschreibung der Abbildungen

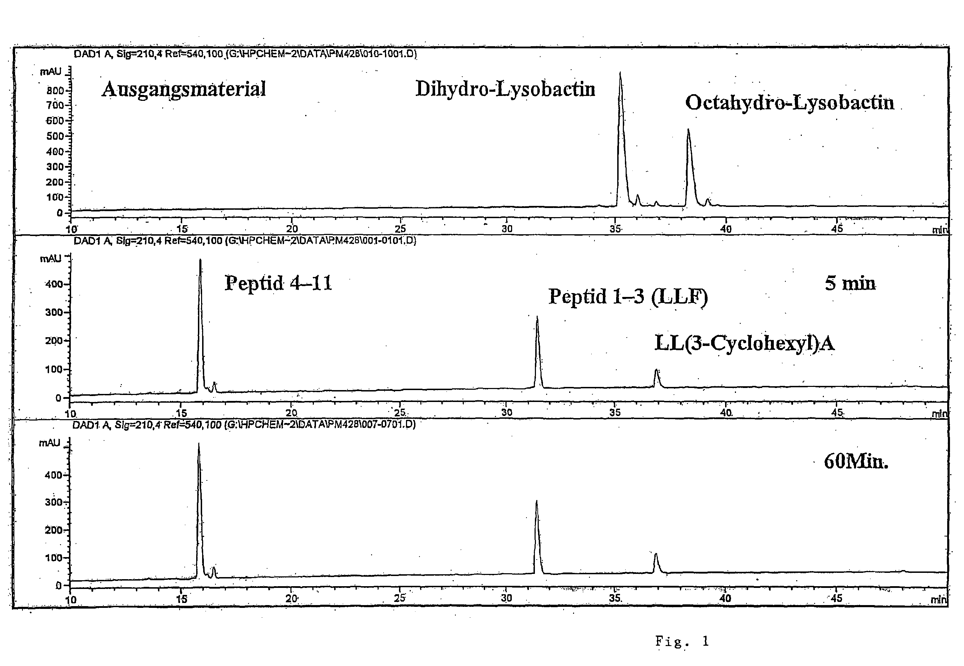

**Abb. 1****:** Zeitlicher Verlauf einer präparativen enzymatischen Spaltung mit Chymotrypsin (Beispiel 11). Überlagerung von HPLC-Diagrammen einer präparativen enzymatischen Spaltung mit Chymotrypsin eines Gemisches aus Dihydro- und Octahydro-Lysobactin. Die Trennbedingungen sind wie in der Beschreibung unter Beispiel 30 angegeben (UV-Detektion 210 nm).

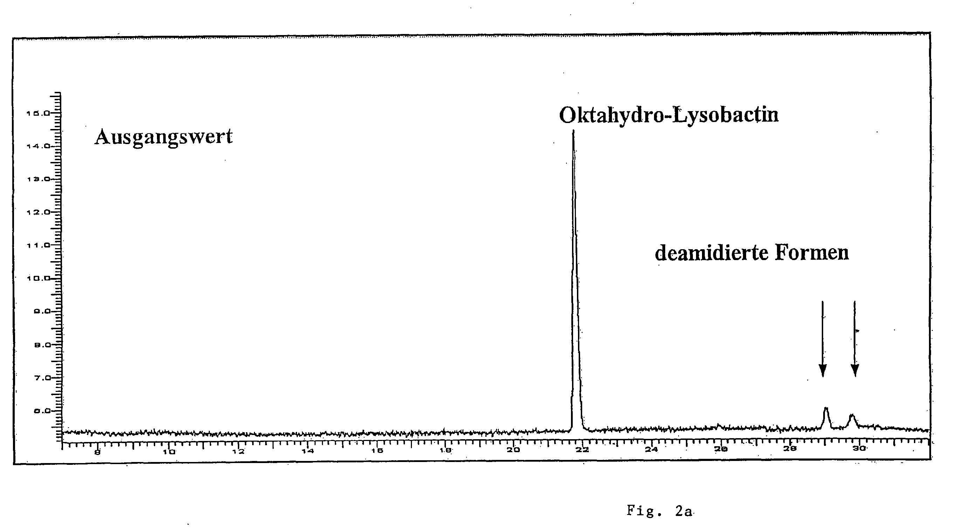

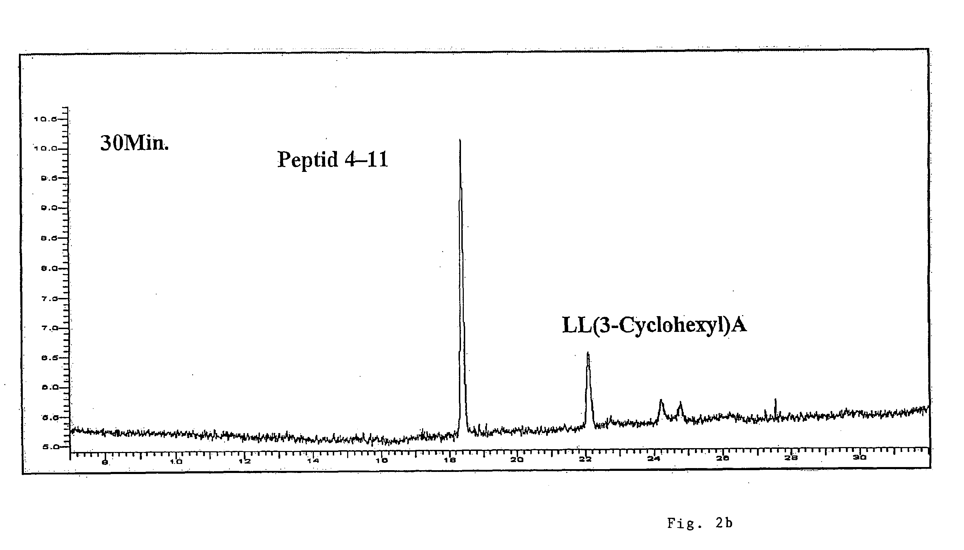

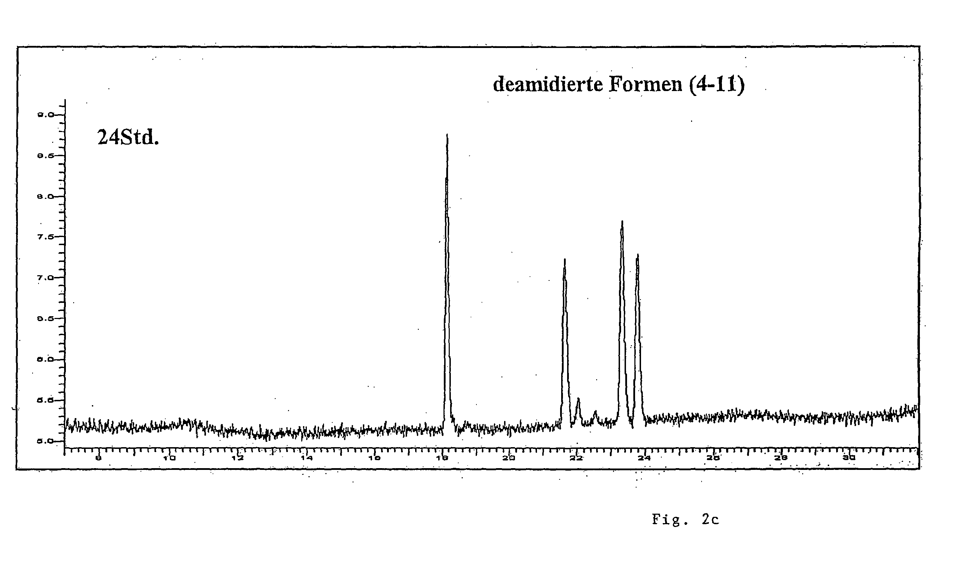

**Abb. 2****:** Zeitlicher Verlauf einer enzymatischen Spaltung von Octahydro-Lysobactin mit Chymotrypsin (Beispiel 5). Überlagerung von CZE-Diagrammen einer enzymatischen Spaltung mit Chymotrypsin von Octahydro-Lysobactin. Die Trennbedingungen sind wie in der Beschreibung unter Beispiel 31 angegeben (UV-Detektion 210 nm).

### Definitionen

Dihydro-Lysobactin: D-Leu-Leu-Phe-Leu(OH)-Leu-D-Arg-Ile-allo-Thr-Gly-Asn(OH)-Ser
Octahydro-Lysobactin: D-Leu-Leu-Ala(3-cyclohexyl)-Leu(OH)-Leu-D-Arg-Ile-allo-Thr-Gly-Asn(OH)-Ser
Lysobactinfragment 4-11: Leu(OH)-Leu-D-Arg-Ile-allo-Thr-Gly-Asn(OH)-Ser
Lysobactinfragment 1-3: D-Leu-Leu-Phe oder D-Leu-Leu-Ala(3-cyclohexyl)
Nachstehend werden die im Verlaufe der chemischen und enzymatischen Reaktionen und analytischen Charakterisierungen eingesetzten Methoden aufgeführt.

### Beispiele

### Abkürzungen

- atm: Atmosphäre (Druckeinheit)
- Bsp.: Beispiel
- CZE: Kapillarzonenelektrophorese
- DCI: direkte chemische Ionisation (bei MS)
- DCM: Dichlormethan
- DMSO: Dimethylsulfoxid
- d. Th.: der Theorie
- EDTA: Ethylendiamintetraessigsäure (Ethylene Diamine Tetraacetic Acid)
- EI: Elektronenstoß-Ionisation (bei MS)
- ESI: Elektrospray-Ionisation (bei MS)
- Fa.: Firma
- h: Stunde(n)
- HPLC: Hochdruck-, Hochleistungsflüssigchromatographie
- HR: High Resolution (Hochauflösung)
- konz.: konzentriert
- LC-MS: Flüssigchromatographie-gekoppelte Massenspektroskopie
- LLF: D-Leu-Leu-Phe
- LL(3-Cyclohexyl)A: D-Leu-Leu-(3-Cyclohexyl)Ala
- min: Minute/Minuten
- MS: Massenspektroskopie
- neg.: negativ
- NMR: Kernresonanzspektroskopie (nuclear magnetic resonance)
- Pd: Palladium
- Pd-C: Palladium auf Kohle
- pos.: positiv
- proz.: Prozentig
- PTFE: Polytetrafluorethylen
- quant.: quantitativ
- RP-HPLC: Reverse Phase HPLC
- RT: Raumtemperatur
- Rₜ: Retentionszeit (bei HPLC)
- TFA: Trifluoressigsäure
- TOF: Flugzeit (time of flight)
- UV: Ultraviolett
- Vis: sichtbar (visible)
- wässr.: wässrig(e)

### Literatur

Zur Nomenklatur der Peptide und Cyclodepsipeptide vergleiche:
1. A Guide to IUPAC Nomenclature of Organic Compounds (Recommendations 1993), 1993, Blackwell Scientific publications.
2. Nomenclature and symbolism for amino acids and peptides. Recommendations 1983. IUPAC-IUB Joint Commission on Biochemical Nomenclature, UK. Biochemical Journal 1984, 219, 345-373. Sowie zitierte Literatur.

### Allgemeine Methoden LC-MS, HR-MS und HPLC

**Methode 1 (LC-MS):** Gerätetyp MS: Micromass ZQ; Gerätetyp HPLC: HP 1100 Series; UV DAD; Säule: Phenomenex Synergi 2 µ Hydro-RP Mercury 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min 2 ml/min; Ofen: 50°C; UV-Detektion: 210 nm.

**Methode 2 (präparative HPLC; Symmetry; Trifluoressigsäure):** Gerät: Gilson Abimed HPLC; UV Detektor 210 nm; binäres Pumpensystem; Säule: SymmetryPrep^{™}C₁₈, Fa. Waters, 7 µm; 300 x 19 mm; Eluent A: 0.05% Trifluoressigsäure in Wasser, Eluent B: 0.05% Trifluoroessigsäure in Acetonitril; Gradient: 0-5 min 5% B mit Flussrate 20 ml/min, 5-30 min Gradientrampe von 5 bis 60% B mit folgender Flussratenerhöhungen: 22 ml/min ab 6 min, 23 ml/min ab 10 min, 24 ml/min ab 15 min; 30-35 min Gradientrampe von 60% bis 98% B mit Flussrateverminderung zu 21 ml/min ab 38 min; 40-45 min 10% B.

**Methode 3 (Methode zur präparativen Trennung von Dihydro- und Octahydro-Lysobactin durch HPLC):** Säule: SymmetryPrep^{™}C₁₈, Fa. Waters, 7 µm 300 x 19 mm; Fluss 25 ml/min; RT; Eluent A: 0.2% TFA in Wasser, Eluent B: Acetonitril, 0-10 min Gradient: 80% A, 20% B bis 35% A, 65% B; 10.01-15 min: 80% A, 20% B; Detektion 210 nm. Fraktionen mittels LC-MS (Methode 1) kontrolliert, am Rotationsverdampfer von Acetonitril befreit und lyophilisiert.

**Methode 4 (Analytische HPLC 1100, ZQ2, Phenomenex, Synergi, Hydro-RP):** Gerätetyp HPLC: HP 1100 Series; UV DAD; Säule: Fa. Phenomenex, MercuryMS, Synergi 2 µ Hydro-RP 20 x 4 mm; Eluent A: Wasser/0.05% Ameisensäure, Eluent B: Acetonitril; Gradient: 0.0-2.5 min, 90-30% A, Fluss 1-2 ml/min, 2.5-3.0 min, 30-5% A, Fluss 2.0 ml/min, 3.0-4.5 min, 5% A; Ofen: 50°C; UV-Detektion: 210 nm.

**Methode 5 (TOF-HR-MS):** TOF-HR-MS-ESI+ Spektren werden mit einem Micromass LCT Gerät (Kapillarspannung: 3.2 KV, Cone-Spannung: 42 V, Quellentemperatur: 120°C, Desolvations-Temperatur: 280°C) aufgenommen. Hierzu wird eine Spritzenpumpe (Fa. Harvard Apparatus) für die Probenzuführung verwendet. Als Standart dient Leucin-Enkephalin (Tyr-Gly-Gly-Phe-Leu).

**Methode 6 (HPLC):** Gerätetyp HPLC: HP 1100 Series; UV DAD Säule: Zorbax Eclipse XBD-C8 (Agilent), 150 mm x 4.6 mm, 5 µm; Eluent A: 5 ml HClO₄/l Wasser, Eluent B: Acetonitril; Gradient: 0-1 min 10% B, 1-4 min 10-90% B, 4-5 min 90% B; Fluss: 2.0 ml/min; Ofen: 30°C; UV-Detektion: 210 und 254 nm.

**Methode 7 (HPLC):** Säule: Kromasil RP-18, 60 mm x 2 mm, 3.5 µm; Eluent A: 5 ml HClO₄/l Wasser, Eluent B: Acetonitril; Gradient: 0 min 2% B, 0.5 min 2% B, 4.5 min 90% B, 9 min 90% B; Fluss: 0.75 ml/min; Ofen: 30°C; UV-Detektion: 210 nm.

**Methode 8 (HPLC):** Säule: Kromasil RP-18, 250 mm x 4 mm, 5 µm; Eluent A: 5 ml HClO₄/l Wasser, Eluent B: Acetonitril; Gradient: 0 min 5% B, 10 min 95% B; Fluss: 1 ml/min; Ofen: 40°C; UV-Detektion: 210 nm.

**Methode 9 (HPLC):** Säule: Kromasil RP-18, 250 mm x 4 mm, 5 µm; Eluent A: 2 ml HClO₄/l Wasser, Eluent B: Acetonitril; Isokratisch: 45% B, 55% A; Fluss: 1 ml/min; Ofen: 40°C; UV-Detektion: 210 nm.

**Methode 10 (HPLC):** Gerät: Agilent 1100 mit DAD (G1315B), binärer Pumpe (G1312A), Autosampler (G1313A), Lösemittel-Entgaser (G1379A) und Säulenthermostat (G1316A); Säule: Agilent Eclipse XDB-C8 4.6 x 150 x 5 mm; Säulentemperatur: 40°C; Eluent A: 0.05% von 70% Perchlorsäure in Wasser; Eluent B: Methanol; Fluss: 2.00 ml/min; Isokratisch: 0-7 min 55% B.

**Methode 11 (HPLC):** Analytische HPLC-Methode Bromelain- / Chymotrypsinspaltung. Ca.20 µg der enzymatischen Spaltprodukte bzw. der Ausgangsverbindungen werden an einer 300SB-C18-column (4.6 mm x 125 mm; 3.5 µm Material; 300 Angström Porendurchmesser) chromatographiert. Als Eluent wird ein Acetonitril / TFA-Gradient verwendet. Eluent A: 0.1% TFA in Wasser, Eluent B: 0.1% TFA in 60% Acetonitril/40% Wasser; Gradient: 0 min 0% B, 2 min 10% B, 50 min 80% B, 52 min 100% B, 55 min 0% B, 60 min 0% B; Fluss: 0.7 ml/min; Säulentemperatur: 40°C; Detektion: 210 nm.

### Proteinchemische Charakterisierung von Dihydro-, Octahydro-Lysobactin und der enzymatischen Spaltprodukte

### Geräte

Die Sequenzanalysen werden mit einen Proteinsequenzer Procise^{™} der Fa. Applied Biosystems durchgeführt. Das Standardsequenzierungsprogramm wird verwendet. Der Sequenzer, die verschiedenen Sequenzierungsprogramme sowie das PTH-Detektionssystem sind im Detail im Bedienungshandbuch beschrieben User's Manual Set, Protein Sequencing System Procise^{™} (1994), Applied Biosystems Forster City, CA 94404, U.S.A.

Die Reagenzien für den Betrieb des Sequenzers und die HPLC-Säule für die PTH-Detektion werden von Applied Biosystems bezogen.

Die HPLC-Analysen werden mit einem HP1100 HPLC-System von Agilent durchgeführt. Eine Zorbax 300SB-C18-column (4.6 mm x 150 mm; 3.5 µm Material; 300 Angström Porendurchmesser) von Agilent (D-Waldbronn) wird für die Trennungen verwendet.

Die verwendeten Reagenzien sind von HPLC-Qualität und werden von Merck (D-Darmstadt) bezogen.

Die Kapillarelektrophorese Modell 270A-HT ist von Applied Biosystems. Die Proben werden in der Regel hydrodynamisch über verschiedene Zeitintervalle injiziert. Die verwendete Kapillarsäule (50 µm Durchmessser x 72 cm Länge) ist von Applied Biosystems. Trennprogramme und die Funktion des Analysators sind ausführlich im Handbuch des Gerätes beschrieben (User's manual capillary electrophoresis system model 270A HT; Applied Biosystems Forster City, CA 94404, U.S.A.; 1989).

Die verwendeten Reagenzien sind von biochemischer Qualität und werden von Merck (D-Darmstadt) oder Sigma (D-Deisenhofen) bezogen.

Die Aminosäureanalysen werden mit einem Aminosäureanalysator LC3000 von Eppendorf / Biotronik durchgeführt. Ein leicht modifiziertes Standardtrennprogramm von Eppendorf / Biotronik wird benutzt. Die Trennprogramme und die Funktion des Analysators sind ausführlich im Handbuch des Gerätes beschrieben (Handbuch des Aminosäureanalysators LC 3000, Wissenschaftliche Geräte GmbH Biotronik, Maintal, 1996).

Die verwendeten Reagenzien sind von biochemischer Qualität und werden von Merck (D-Darmstadt), Fluka (D-Neu-Ulm) oder Sigma (D-Deisenhofen) bezogen.

Die Molekulargewichte werden mit einem ZQ-1 System von Micromass (Manchester, UK) bestimmt. Die Fragmente werden dabei mittels RP-18-HPLC-Chromatographie (HP1100-System) getrennt und das Molekulargewicht durch Elektronenspray-Ionisation (ESI) bestimmt. Eine externe Kalibrierung wird durchgeführt. Die Kalibrierung und Funktion des Systems sind ausführlich im Handbuch des Gerätes beschrieben.

Die eingesetzten Enzyme und Chemikalien sind von biochemischer Qualität und werden von den Firmen Fluka, Calbiochem (D-Heidelberg) und Sigma bezogen.

Das Material für die präparative Chromatographie Source 15RPC wird von Amersham Bioscience (D-Freiburg) bezogen. Die präparative Trennung wird mit einem ÄKTA^{™}-System von Amersham Bioscience durchgeführt.

Die in der Erfindung erwähnten chemischen Verbindungen können auch in Form von Salzen, Solvaten oder Solvaten der Salze vorliegen.

Als Salze sind im Rahmen der vorliegenden Erfindung physiologisch unbedenkliche Salze der erfindungsgemäß herstell- oder verwendbaren Verbindungen bevorzugt. Umfasst sind aber auch Salze, die für pharmazeutische Anwendungen selbst nicht geeignet sind aber beispielsweise für die Isolierung oder Reinigung der erfindungsgemäß herstell- oder verwendbaren Verbindungen verwendet werden können oder Mischsalze.

Physiologisch unbedenkliche Salze der erfindungsgemäß herstell- oder verwendbaren Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäß herstell- oder verwendbaren Verbindungen umfassen auch Salze üblicher Basen, wie beispielhaft und vorzugsweise Alkalimetallsalze (z.B. Natrium-und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäß herstell- oder verwendbaren Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

### Beispiel 1

D-Leucyl-N¹-{(3S,6S,12S,15S,18R,21S,24S,27S,28R)-6-[(1S)-2-amino-1-hydroxy-2-oxoethyl]-18-(3-{[amino(imino)methyl]amino}propyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclooctacosan-27-yl}-L-leucinamid-bistrifluoracetat

{D-Leucyl-L-leucyl-[(3R)-3-hydroxy-L-phenylalanyl)]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.11}-O^{3.3}-lacton-bistrifluoracetat} (Lysobactin)

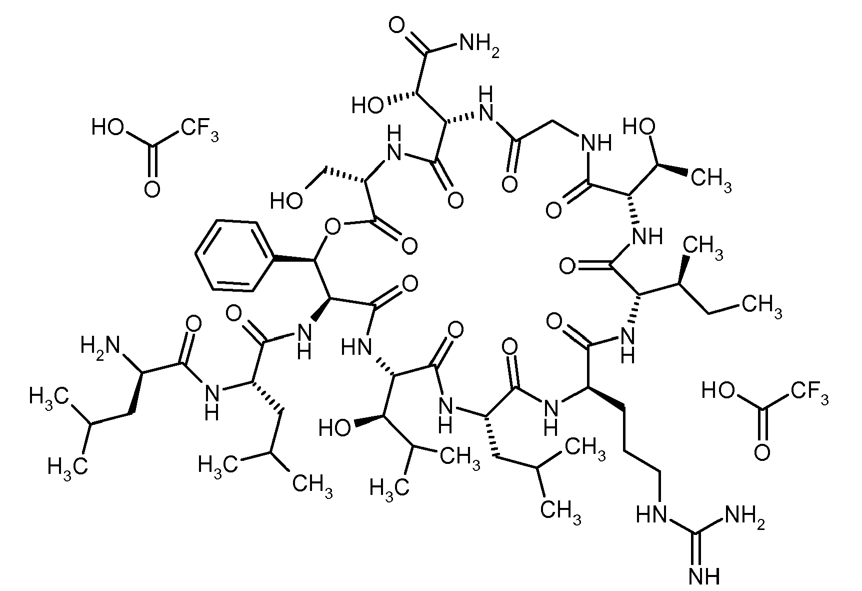

### Fermentation:

### Kultur Medium:

YM: Hefe-Malz Agar: D-Glucose (4 g/l), Hefe Extrakt (4 g/l), Malz Extrakt (10 g/l), 1 Liter Lewatit Wasser. Vor dem Sterilisieren (20 Minuten bei 121°C) wird der pH auf 7.2 eingestellt.

HPM: Mannit (5.4 g/l), Hefe Extrakt (5 g/l), Fleischpepton (3 g/l).

Arbeitskonserve: Der lyophilisierte Stamm (ATCC 53042) wird in 50 ml YM Medium angezogen.

Kolbenfermentation: 150 ml YM Medium oder 100 ml HPM Medium in einem 1 l Erlenmeyerkolben werden mit 2 ml der Arbeitskonserve beimpft und für 30-48 Stunden bei 28°C auf einem Schüttler bei 240 Upm wachsen gelassen.

30 1 Fermentation: 300 ml der Kolbenfermentation (HPM Medium) werden zum Animpfen einer sterilen 30 1 Nährmedienlösung verwandt (1 ml Antifoam SAG 5693/1). Diese Kultur wird für 21 Stunden bei 28°C, 300 Upm und einer Belüftung mit steriler Luft von 0.3 vvm wachsen gelassen. Der pH wird mit 1 M Salzsäure auf pH = 7.2 konstant gehalten. Insgesamt werden während der Kultivierungszeit 880 ml 1 M Salzsäure zugeführt.

Hauptkultur (200 l): 15 x 150 ml YM Medium in 1 l Erlenmeyerkolben werden mit 2 ml der Arbeitskonserve beimpft und bei 28°C für 48 Stunden und 240 Upm auf dem Schüttler wachsen gelassen. 2250 ml dieser Kultur werden zum Beimpfen einer sterilen 200 l Nährmedienlösung (YM) verwandt (1 ml Antifoam SAG 5693/1) und für 18.5 Stunden bei 28°C, 150 Upm und einer Belüftung mit steriler Luft von 0.3 vvm wachsen gelassen.

Zur Kontrolle des Fermentationsverlaufs werden stündlich Proben (50 ml) entnommen. 2 ml dieser Kulturbrühe werden mit 1 ml Methanol (0.5% Trifluoressigsäure) versetzt und über einen 0.45 µm Filter filtriert. 30 µl dieser Suspension werden mittels HPLC analysiert (Methode 6 und Methode 7).

Nach 18.5 Stunden wird die Kulturbrühe der Hauptkultur bei 17000 Upm in Überstand und Sediment getrennt.

### Isolierung:

Der Überstand (183 l) wird mit konzentrierter Trifluoressigsäure bzw. Natronlauge auf pH 6.5 bis 7 eingestellt und auf eine Lewapolsäule (OC 1064, 60 l Inhalt) aufgetragen. Anschließend wird mit reinem Wasser, Wasser/Methanol 1:1 und anschließend mit reinem Methanol (mit 0.1% Trifluoressigsäure) eluiert. Diese organische Phase wird im Vakuum bis zu einem verbleibenden wässrigen Rest von 11.5 l eingeengt.

Die verbleibende wässrige Phase wird an Kieselgel C₁₈ gebunden und aufgetrennt (MPLC, Biotage Flash 75, 75 x 30 cm, KP-C18-WP, 15-20 µm, Fluss: 30 ml; Eluent: Acetonitril/ Wasser mit 0.1% Trifluoressigsäure; Gradient: 10%, 15% and 40% Acetonitril). Die 40% Acetonitril Phase, die die Hauptmenge von Beispiel 1A enthält, wird im Vakuum eingeengt und anschließend lyophilisiert (ca. 13 g). Dieses Feststoffgemisch wird in 1.2 g Portionen zunächst an einer präparativen HPLC (Methode 1), anschließend durch Gelfiltration an Sephadex LH-20 (5 x 70 cm, Acetonitril/Wasser 1:1, jeweils mit 0.05% Trifluoressigsäure) und einer weiteren präparativen HPLC (Methode 8) getrennt.

Dieser Prozess liefert 2250 mg Beispiel 1.

Das Sediment wird in 4 l Aceton/Wasser 4:1 aufgenommen, mit 2 kg Celite versetzt, mit Trifluoressigsäure auf pH = 6 eingestellt, ausgerührt und zentrifugiert. Das Lösungsmittel wird im Vakuum eingeengt und der Rückstand gefriergetrocknet. Das erhaltene Lyophilisat (89.9 g) wird in Methanol aufgenommen, abfiltriert, eingeengt und an Kieselgel (Methode 9) getrennt. Beispiel 1A wird danach per Gelfiltration (Sephadex LH-20, 5 x 68 cm, Wasser/Acetonitril 9:1 (mit 0.05% Trifluoressigsäure), Fluss: 2.7 ml/min, Fraktionsgröße 13.5 ml) zur Reinsubstanz aufgereinigt.

Dieser Prozess liefert 447 mg Beispiel 1.

### HPLC (Methode 6): Rₜ = 6.19 min

MS (ESIpos): m/z = 1277 [M + H]⁺

¹H NMR (500.13 MHz, d₆-DMSO): δ = 0.75 (d, 3H), 0.78 (d, 6H), 0.80 (t, 3H), 0.82 (d, 3H), 0.90 (d, 3H), 0.91 (d, 3H), 0.92 (d, 3H), 0.95 (d, 3H), 0.96 (d, 3H), 1.05 (m, 1H), 1.19 (d, 3H), 1.25 (m, 2H), 1.50 (m, 4H), 1.51 (m, 2H), 1.55 (m, 1H), 1.61 (m, 1H), 1.65 (m, 1H), 1.84 (m, 1H), 1.85 (m, 1H), 1.86 (m, 1H), 1.89 (m, 1H), 1.95 (m, 1H), 2.75 (m, 2H), 3.40 (m, 1H), 3.52 (m, 2H), 3.53 (dd, 1H), 3.64 (m, 2H), 3.66 (m, 1H), 3.68 (dd, 1H), 3.73 (m, 2H), 4.00 (dd, 1H), 4.02 (br., 1H), 4.13 (br., 1H), 4.32 (dd, 1H), 4.39 (t, 1H), 4.55 (m, 1H), 4.75 (dd, 1H), 5.19 (t, 1H), 5.29 (d, 1H), 5.30 (br., 1H), 5.58 (m, 2H), 6.68 (m, 3H), 6.89 (d, 1H), 6.93 (m, 3H), 6.94 (br., 1H), 6.98 (d, 1H), 7.12 (br., 1H), 7.20 (br., 2H), 7.23 (m, 2H), 7.42 (m, 2H), 7.54 (d, 1H), 7.58 (d, 1H), 8.32 (br., 1H), 9.18 (br., 1H), 9.20 (m, 2H), 9.50 (br., 1H).

¹³C-NMR (125.77 MHz, d₆-DMSO): δ = 10.3, 15.3, 19.0, 19.2, 19.6, 20.0, 20.9, 22.0, 22.4, 23.0, 23.2, 24.3, 24.4, 25.0, 25.4, 26.0, 27.8, 30.9, 35.4, 39.5, 40.8, 40.9, 41.6, 44.1, 51.5, 52.7, 55.9, 56.2, 56.4, 57.9, 58.8, 60.2, 61.1, 62.6, 70.1, 71.6, 71.7, 75.5, 128.1, 128.6, 136.7, 156.8, 168.2, 170.1, 170.4, 171.2, 171.5, 171.9, 172.2, 172.4, 173.7.

Die Zuordnung der Signale erfolgte nach der in der Literatur beschriebenen Zuordnung (T. Kato, H. Hinoo, Y. Terui, J. Antibiot., 1988, 61, 719-725).

### Beispiel 2 und Beispiel 3

D-Leu-Leu-Phe-[(3R)-Leu(3-OH)]-Leu-D-Arg-Ile-aThr-Gly-[(3S)-3-Asn(3-OH)]-Ser-trifluoracetat (Dihydro-Lysobactin) und

D-Leu-Leu-Ala(3-cyclohexyl)-[(3R)-Leu(3-OH)]-Leu-D-Arg-Ile-aThr-Gly-[(3S)-3-Asn(3-OH)]-Ser-trifluoracetat (Octahydro-Lysobactin)

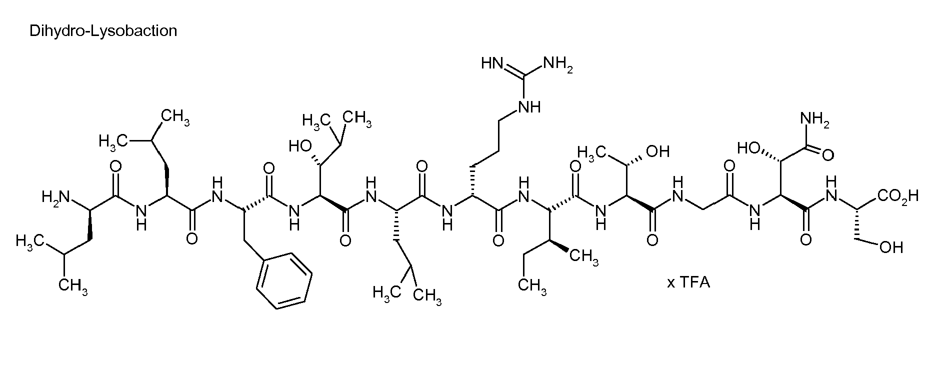

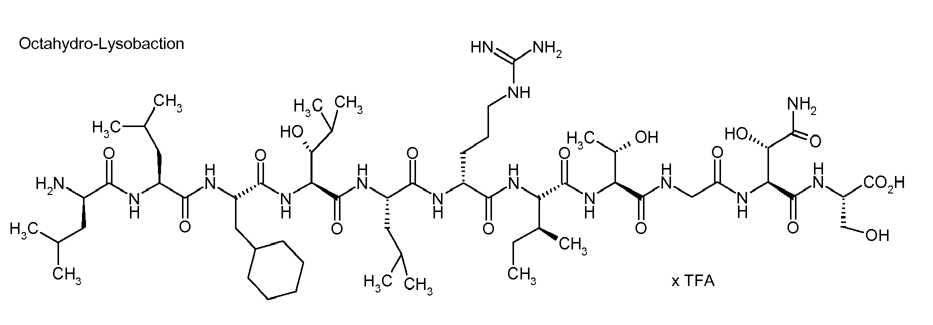

### Hydrierverfahren 1:

Die Verbindung aus Beispiel 1 (Lysobactin, 250 mg, 170 µmol) wird in Isopropanol/Wasser (2:1, 60 ml) gelöst und in Gegenwart von 200 mg Pd (10% auf Kohle) unter 1 atm Wasserstoff hydriert. Der Verlauf der Reaktion wird mittels LC-MS (Methode 1) verfolgt. Nach nahezu vollständigem Umsatz (>95%) wird der Katalysator abfiltriert, mit Isopropanol gewaschen und das Filtrat lyophylisiert. In diesem Rohprodukt verteilen sich laut LC-MS die Produkte wie folgt: Dihydro-Lysobactin ca. 74%, Octahydro-Lysobactin ca. 12%. Der Rückstand wird durch HPLC (Methode 2) gereinigt. Nach Lyophilisierung der geeigneten Fraktionen erhält man die reine Verbindung Beispiel 2 (81.5 mg, 31% d. Th.).

LC-MS: (Methode 1): Rₜ= 1.56 min ES⁺: m/z = 1279 [M + H]⁺, 640.1 [M + 2H]²⁺; ES-: m/z = 1277 [M - H]⁻, 638.1 [M - 2H]²⁻.

Peptidsequenzen der Hydro-Lysobactine siehe Tabelle 1.

### Hydrierverfahren 2:

Mit Hydrierung unter Wasserstoffdruck von 3 atm erhält man in dem sonst mit Hydrierverfahren 1 identischen Verfahren die durch LC-MS bestimmte folgende Verteilung im Rohprodukt: Dihydro-Lysobactin ca. 80%, Octahydro-Lysobactin ca. 17%. Nach HPLC-Reinigung (Methode 2) erhält man die reine Verbindung Beispiel 2 (86 mg, 33% d. Th).

### Hydrierverfahren 3:

Bei verlängerter Hydrierungsdauer bei 3 bar Wasserstoff oder mit mehr Druck (bis 80 bar Wasserstoffdruck) kann man anteilig mehr Octahydro-Lysobactin erhalten. In den meisten Fällen werden die Rohmischungen von Dihydro- und Octahydro-Lysobactin nicht getrennt, sondern direkt in die enzymatische Spaltung eingesetzt.

### Hydrierverfahren 4:

Im folgendem Fall wird die Verbindung Octahydro-Lysobactin auch in reiner Form isoliert:

Lysobactin (Beispiel 1, 1.04 g, 0.69 mmol) wird in Isopropanol/Wasser (2:1, 90 ml) gelöst und in Gegenwart von 200 mg Pd (10% auf Kohle) unter 3 atm Wasserstoff für 7 Tagen hydriert. Der Katalysator wird abfiltriert, mit Isopropanol gewaschen und das Filtrat am Rotationsverdampfer vom Isopropanol befreit und dann lyophylisiert. In diesem Rohprodukt verteilen sich laut LC-MS (Methode 1) die Produkte wie folgt: Dihydro-Lysobactin ca. 65%, Octahydro-Lysobactin ca. 35%. Der Rückstand wird durch HPLC (Methode 2, anschließend Methode 3) gereinigt. Man erhält Dihydro-Lysobactin (Beispiel 2) (280 mg, 27% d. Th.) und Octahydro-Lysobactin (Beispiel 3) (212 mg, 20% d. Th.).

LC-MS: (Methode 1): Rₜ = 1.63 min ESIpos.: m/z = 643.3 (100) [M + 2H]²⁺; ESIneg.: m/z = 1283 [M - H]⁻, 641.2 [M - 2H]²⁻.

### Hydrierverfahren 5:

Als Beispiel für eine Hydrierung bei hohem Druck Wasserstoff erhält man nach 4 Tagen bei 40°C und 50 bar Wasserstoff die folgende Rohmischung laut LC-MS (Methode 1): 45% Dihydro-Lysobactin und 45% Octahydro-Lysobactin.

### Hydrierverfahren 6:

Lysobactin-bistrifluoracetat (Beispiel 1, 500 mg, 0.33 mmol) wird in Isopropanol/Wasser 2:1 (30 ml) gelöst. Unter Argonschutzgasatmosphäre wird 10proz. Palladium auf Kohle (100 mg) zugegeben. Das Reaktionsgemisch wird (nach Entgasung) in einem Druckautoklaven bei 80-70 bar Wasserstoff und RT für 48 h gerührt. Zur Reaktion wird erneut 10proz. Palladium auf Kohle (100 mg) gegeben. Das Reaktionsgemisch wird (nach Entgasung) erneut in einem Druckautoklaven bei 80-70 bar Wasserstoff und RT für 48 h gerührt. Nun ist mittels HPLC (z. B. Methode 4) kein Lysobactin mehr detektierbar. Das Reaktionsgemisch wird über einer Glasfritte (Porengröße 2 oder 3) filtriert, im Vakuum eingeengt, erneut in Methanol/0.2% Eisessig aufgenommen, über einem Spritzenfilter (Fa. Biotage, PTFE) filtriert, im Vakuum eingeengt und im Hochvakuum getrocknet. Man erhält 496 mg (quant.) Produkt (80% Dihydro-Lysobactin, 20% Octahydro-Lysobactin).

### Hydrierverfahren 7:

Lysobactin-Monotrifluoracetat-monoacetat (5 mg, 3.45 µmol) wird in einer Mischung von Isopropanol (2 ml), Wasser (0.25 ml) und Essigsäure (0.05 ml) in Gegenwart von Platindioxid (20 mg) bei 80 bar und 50°C hydriert. Nach 17 h wird der Druck ausgelassen, mit Argon gelüftet und die Suspension durch Mikrofilter vom Katalysator befreit. LC-MS-Analyse des Filtrats (Methode 4) zeigt 7% d. Th. Octahydro-Lysobactin (Rₜ = 1.54 min, Methode 4).

### Hydrierverfahren 8:

Lysobactin-bistrifluoracetat (Beispiel 1A, 10 g, 6.65 mmol) wird in Isopropanol/Wasser 9:2 (110 ml) gelöst. Unter Argonschutzgasatmosphäre wird Palladium auf Kohle (10%ig; 5 g) zugegeben. Das Reaktionsgemisch wird (nach Entgasung) in einem Druckautoklaven bei 80-70 bar Wasserstoffdruck und 40°C für 12 h gerührt. Zur Reaktion wird erneut Palladium auf Kohle (10%ig; 5 g) gegeben. Das Reaktionsgemisch wird (nach Entgasung) erneut in einem Druckautoklaven bei 80-70 bar Wasserstoffdruck und 40°C für 12 h gerührt. Das Reaktionsgemisch wird (nach Entgasung) wieder einmal in einem Druckautoklaven bei 80-70 bar Wasserstoffdruck und 40°C für 12 h gerührt. Nun ist mittels analytischer HPLC (Methode 10) kein Lysobactin mehr detektierbar. Das Reaktionsgemisch wird über Kieselgur filtriert, im Vakuum eingeengt und im Hochvakuum getrocknet. Man erhält 9.17 g (99% d. Th.) Produkt (60% Dihydrolysobactin, 40% Octahydrolysobactin).

### Hydrierverfahren 9:

Lysobactin-bistrifluoracetat (Beispiel 1A, 5 g, 3.32 mmol) wird in Isopropanol/Wasser 9:2 (110 ml) gelöst. Unter Argonschutzgasatmosphäre wird Palladium auf Kohle (10%ig; 5 g) zugegeben. Das Reaktionsgemisch wird (nach Entgasung) in einem Druckautoklaven bei 80 bar Wasserstoffdruck und 40°C für 12 h gerührt. Das Reaktionsgemisch wird über Kieselguhr filtriert, im Vakuum eingeengt und im Hochvakuum getrocknet. Die Hydrierung wird noch dreimal mit je 5.0 g Lysobactin-bistrifluoracetat wiederholt (gesamt: 4 Durchgänge). Man erhält als vereinigte Produktfraktion 18.27 g Produkt (Dihydrolysobactin:Octahydrolysobactin, ca. 5:4).

### Beispiel 4

### Chymotrypsinspaltung von Dihydro-Lysobactin Verhältnis Enzym / Substrat 1:50

200 µg Dihydro-Lysobactin werden in 10 µl Methanol gelöst und dann mit 190 µl Spaltpuffer (0.1 M Ammoniumhydrogencarbonat / 0.5 M Harnstoff pH 8) versetzt. 4 µg Chymotrypsin (1:50) werden hinzugegeben und die Reaktion bei 37°C durchgeführt. Es werden 30 µl Aliquots nach 0, 0.5, 1, 3, 6 und 24 h entnommen und die Enzymspaltung mit 30 µl Acetonitril / 1% TFA abgestoppt. Die Proben werden bis zur Analyse mit der HPLC, der Kapillarzonenelektrophorese, der Sequenzanalyse, der Aminosäureanalyse oder MS-Untersuchung bei -20°C gelagert.

Peptidsequenzen der Chymotrypsin-Spaltprodukte siehe Tabelle 2.

### Beispiel 5

### Chymotrypsinspaltung von Octahydro-Lysobactin Verhältnis Enzym / Substrat 1:50

200 µg Octahydro-Lysobactin werden in 10 µl Methanol gelöst und dann mit 190 µl Spaltpuffer (0.1 M Ammoniumhydrogencarbonat / 0.5 M Harnstoff pH 8) versetzt. 4 µg Chymotrypsin (1:50) werden hinzugegeben und die Reaktion bei 37°C durchgeführt. Es werden 30 µl Aliquots nach 0, 0.5, 1, 3, 6 und 24 h entnommen und die Enzymspaltung mit 30 µl Acetonitril / 1% TFA abgestoppt. Die Proben werden bis zur Analyse bei -20°C gelagert.

Peptidsequenzen der Chymotrypsin-Spaltprodukte siehe Tabelle 2.

### Beispiel 6

### Analytische Chymotrypsinspaltung vom Gemisch Dihydro- / Octahydro-Lysobactin Verhältnis Enzym / Substrat 1:25

200 µg Dihydro- (59%) und Octahydro-Lysobactin (34%) werden in 10 µl Methanol gelöst und dann mit 190 µl Spaltpuffer (0.1 M Ammoniumhydrogencarbonat / 0.5 M Harnstoff pH 8) versetzt. 8 µg Chymotrypsin (1:25) werden hinzugegeben und die Reaktion bei 37°C durchgeführt. Es werden 30 µl Aliquots nach 0, 0.5, 1, 3 h entnommen und die Enzymspaltung mit 30 µl Acetonitril / 1% TFA abgestoppt. Die Proben werden bis zur Analyse bei -20°C gelagert.

Peptidsequenzen der Chymotrypsin-Spaltprodukte siehe Tabelle 2.

### Beispiel 7

### Analytische Chymotrypsinspaltung vom Gemisch Dihydro- / Octahydro-Lysobactin Verhältnis Enzym / Substrat 1:400

150 µg Dihydro- (59%) und Octahydro-Lysobactin (34%) werden in 15 µl Ethanol gelöst und dann mit 126 µl Spaltpuffer (0.1 M Ammoniumhydrogencarbonat / 0.5 M HarnstoffpH 8) versetzt. 0.38 µg Chymotrypsin (9 µl Chymotrypsinlösung Wasser/ Ethylenglykol / Spaltpuffer, 0.2 mg/ml; 1:400) werden hinzugegeben und die Reaktion bei 37°C durchgeführt. Es werden 25 µl Aliquots nach 0, 0.5, 1, 3 h entnommen und die Enzymspaltung mit 25 µl 30% Acetonitril / 0.1% TFA abgestoppt. Die Proben werden bis zur Analyse bei -20°C gelagert.

Peptidsequenzen der Chymotrypsin-Spaltprodukte siehe Tabelle 2.

### Beispiel 8

### Analytische Chymotrypsinspaltung vom Gemisch Dihydro- / Octahydro-Lysobactin Substratkonzentration 6 mg/ml

900 µg Dihydro- (59%) und Octahydro-Lysobactin (34%) werden in 15 µl Methanol gelöst und dann mit 99 µl Spaltpuffer (0.1 M Ammoniumhydrogencarbonat / 0.5 M Harnstoff pH 8) versetzt. 36 µg Chymotrypsin (36 µl Chymotrypsinlösung Wasser/ Ethylenglykol 1:1, 1 mg/ml; 1:25) werden hinzugegeben und die Reaktion bei 37°C durchgeführt. Es werden 25 µl Aliquots nach 0, 0.5, 1, 3 h entnommen und die Enzymspaltung mit 25 µl 30% Acetonitril / 0.1% TFA abgestoppt. Die Proben werden bis zur Analyse bei -20°C gelagert.

Peptidsequenzen der Chymotrypsin-Spaltprodukte siehe Tabelle 2.

### Beispiel 9

### Analytische Chymotrypsinspaltung vom Gemisch Dihydro- / Octahydro-Lysobactin Lösungsmittelkonzentration 30% Methanol

150 µg Dihydro- (59%) und Octahydro-Lysobactin (34%) werden in 45 µl Methanol gelöst und dann mit 99 µl Spaltpuffer (0.1 M Ammoniumhydrogencarbonat / 0.5 M Harnstoff pH 8) versetzt. 6 µg Chymotrypsin (6 µl Chymotrypsinlösung Wasser/Ethylenglykol 1:1, 1 mg/ml; 1:25) werden hinzugegeben und die Reaktion bei 37°C durchgeführt. Es werden 25 µl Aliquots nach 0, 0.5, 1, 3 h entnommen und die Enzymspaltung mit 25 µl 30% Acetonitril / 0.1% TFA abgestoppt. Die Proben werden bis zur Analyse bei -20°C gelagert.

Peptidsequenzen der Chymotrypsin-Spaltprodukte siehe Tabelle 2.

### Beispiel 10

### Analytische Chymotrypsinspaltung vom Gemisch Dihydro- / Octahydro-Lysobactin Spaltung bei Raumtemperatur

200 µg Dihydro- (59%) und Octahydro-Lysobactin (34%) werden in 10 µl Methanol gelöst und dann mit 190 µl Spaltpuffer (0.1 M Ammoniumhydrogencarbonat / 0.5 M Harnstoff pH 8) versetzt. 8 µg Chymotrypsin (8 µl Chymotrypsinlösung Wasser/ Ethylenglykol 1:1, 1 mg/ml; 1:25) werden hinzugegeben und die Reaktion bei Raumtemperatur (20-25°C) durchgeführt. Es werden 30 µl Aliquots nach 0, 0.5, 1, 3, 6 h entnommen und die Enzymspaltung mit 30 µl 30% Acetonitril / 1% TFA abgestoppt. Die Proben werden bis zur Analyse bei -20°C gelagert.

Peptidsequenzen der Chymotrypsin-Spaltprodukte siehe Tabelle 2.

### Beispiel 11

### Fragment 4-11

[(3R)-Leu(3-OH)]-Leu-D-Arg-Ile-aThr-Gly-[(3S)-3-Asn(3-OH)]-Ser-trifluoracetat

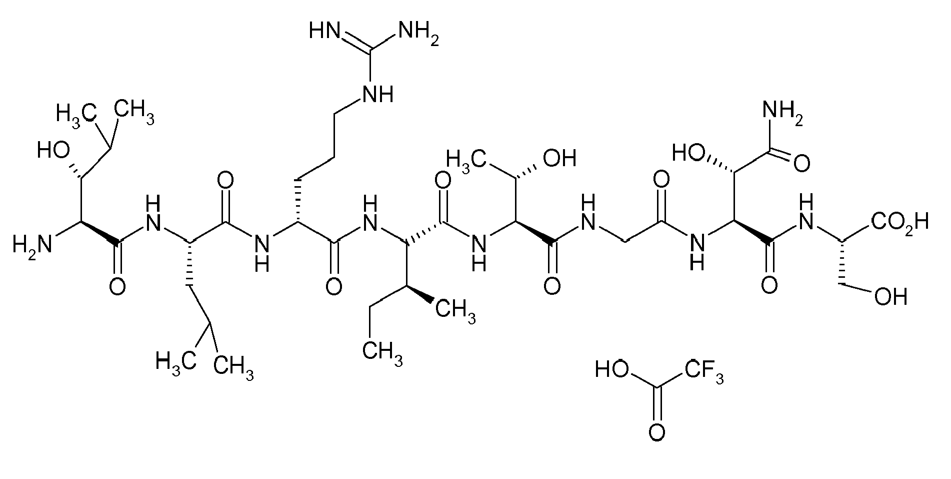

### Präparative Chymotrypsinspaltung vom Dihydro-Lysobactin Substratkonzentration 1 mg/ml

2 x 80 mg Dihydro-Lysobactin (35.3 µmol und 33.8µmol reines Peptid bestimmt durch Aminosäureanalyse) werden in je 8 ml Methanol gelöst und dann mit je 69 ml Spaltpuffer (0.1 M Ammoniumhydrogencarbonat / 0.5 M Harnstoff pH 8) versetzt. Vor der Enzymzugabe werden die Lösungen im Trockenschrank auf 37°C erwärmt. 3.2 mg Chymotrypsin (3.2 ml Chymotrypsinlösung Wasser /Ethylenglykol 1:1, 1 mg/ml; 1:25; 37°C vorgewärmt) werden hinzugegeben und die Reaktionen bei 37°C durchgeführt. Es werden 200 µl Aliquots nach 0.5, 1 h entnommen und die Enzymspaltungen mit 200 µl 30% Acetonitril / 0.1% TFA abgestoppt. Die Proben werden mit der HPLC parallel zu den Enzymspaltungen innerhalb von 15 min analysiert (Retentionszeit Fragment 4-11 ca. 3.6 min, Fragment 1-3 (LLF) ca. 9.6 min, Bedingungen: Solvent A 0.1% TFA, Solvent B 60% Acetonitril/0.1% TFA, Gradient: 0 min 30% B, 10 min 80% B, 11 min 100% B, 12 min 30% B, 15 min 30% B; Fluss 0.7 ml/min, 40°C, UV-Detektion 210 nm). Die Enzymreaktionen werden nach ca. 70 min mit 3 ml Acetonitril und ca. 0.6 ml TFA abgestoppt. Der pH-Wert der Lösung liegt zwischen 1 und 2. Bis zur präparativen Trennung können die Lösungen bei -20°C gelagert werden.

### Präparative Trennung der Fragmente 1-3 und 4 -11

2 x ca. 80 ml der Spaltlösungen werden über einen Filter (0.2 µm) filtriert und dann vereinigt. Die Lösung wird in vier Portionen je ca. 38.5 ml (gesamt 154 ml) aufgeteilt und jeweils an einer Source 15RPC-Säule (3 ml) mit einem Acetonitril/TFA-Gradienten chromatographiert. Bedingungen: Solvent A 0.1% TFA, Solvent B 0.1% TFA/Acetonitril; Gradient: 0% B nach 45% B in 40 min; Fluss 2 ml/ min; UV-Detektion 210 nm. Die vier Läufe werden nacheinander durchgeführt und die Fraktionen im gleichen Röhrchen gesammelt. Die resultierenden Chromatogramme sind deckungsgleich.

Die Fragmente 4-11 (Rₜ = ca. 15 min) und 1-3 (LLF) (Rₜ = ca. 25 min) werden zusammengegeben, mit Wasser 1:1 verdünnt und dann lyophilisiert.

200 µl Aliquots der jeweiligen Pools werden für die Aminosäureanalyse, analytische HPLC, Kapillarzonenelektrophorese (CZE), Sequenzanalyse und Massenspektrometrie getrennt lyophilisiert.

Die Ausbeute an Fragment 4-11 beträgt nach der Aminosäureanalyse 68.3µmol (99% d. Th.) und an Fragment 1-3 67.4µmol (98% d. Th.).

### Beispiel 12

### Präparative Chymotrypsinspaltung vom Gemisch Dihydro- / Octahydro-Lysobactin 1 mg/ml

### Ansatz 1

2 x 700 mg Dihydro- (56%) und Octahydro-Lysobactin (21%) (682 µmol Dihydro- und Octahydro-Lysobactin enthalten als reine Peptide bestimmt durch Aminosäureanalyse) werden in je 70 ml Methanol gelöst und dann mit je 602 ml Spaltpuffer (0.1 M Ammoniumhydrogencarbonat / 0.5 M Harnstoff pH 8) versetzt. Vor der Enzymzugabe werden die Lösungen im Trockenschrank auf 37°C erwärmt. 28 mg Chymotrypsin (28 ml Chymotrypsinlösung Wasser / Ethylenglykol 1:1, 1 mg/ml; 1:25; 37°C vorgewärmt) werden hinzugegeben und die Reaktionen bei 37°C durchgeführt. Es werden 200 µl Aliquots nach 0.5, 1 h entnommen und die Enzymspaltungen mit 200 µl 30% Acetonitril / 0.1% TFA abgestoppt. Die Proben werden mit der HPLC parallel zu den Enzymspaltungen innerhalb von 15 min analysiert (Retentionszeit Fragment 4-11 ca. 3.6 min, Fragment 1-3 (LLF) ca. 9.6 min, Fragment 1-3 (LL(3-Cyclohexyl)A) ca. 11.3 min, Bedingungen: Solvent A 0.1% TFA, Solvent B 60% Acetonitril/0.1% TFA, Gradient: 0 min 30% B, 10 min 80% B, 11 min 100% B, 12 min 30% B, 15 min 30% B; Fluss 0.7 ml/min, 40°C, UV-Detektion 210 nm). Die Enzymreaktionen werden nach ca. 60 min mit 30 ml Acetonitril und ca. 6 ml TFA abgestoppt. Der pH-Wert der Lösung liegt zwischen 1 und 2. Bis zur präparativen Trennung können die Lösungen bei -20°C gelagert werden.

### Ansatz 2

775 mg Dihydro- (45%) und Octahydro-Lysobactin (48%) (468 µmol Dihydro- und Octahydro-Lysobactin enthalten als reine Peptide bestimmt durch Aminosäureanalyse) werden in 77.5 ml Methanol gelöst und dann mit 667 ml Spaltpuffer (0.1M Ammoniumhydrogencarbonat / 0.5M Harnstoff pH 8) versetzt. Vor der Enzymzugabe wird die Lösung im Trockenschrank auf 37°C erwärmt. 31 mg Chymotrypsin (31 ml Chymotrypsinlösung Wasser / Ethylenglykol 1:1, 1mg/ml; 1:25; 37°C vorgewärmt) werden hinzugegeben und die Reaktion bei 37°C durchgeführt. Es werden 200 µl Aliquots nach 0.5, 1 Std. entnommen und die Enzymspaltung mit 200 µl 30% Acetonitril / 0.1% TFA abgestoppt. Die Proben werden mit der HPLC parallel zur Enzymspaltung innerhalb von 15 min. analysiert (Retentionszeit Fragment 4-11 ca. 3.6 min, Fragment 1-3 (LLF) ca. 9.6 min, Fragment 1-3 (LL(3-Cyclohexyl)A) ca. 11.3 min) (Solvent A 0.1% TFA, Solvent B 60% Acetonitril/0.1% TFA, Gradient 0 min 30% B, 10 min 80% B, 11 min 100% B, 12 min 30% B, 15 min 30% B; Fluss: 0.7 ml/min, Temperatur: 40°C, UV-Detektion 210 nm). Die Enzymreaktion wird nach 60 min. mit 30 ml Acetonitril und ca. 6 ml TFA abgestoppt. Der pH-Wert der Lösung soll zwischen 1 und 2 liegen. Bis zur präparativen Trennung kann die Lösung bei -20°C gelagert werden.

### Präparative Trennung der Fragmente 1-3 und 4 -11

Die Spaltansätze 1 und 2 werden über einen Filter (0.2 µm) filtriert und dann vereinigt. Die Lösung wird in mehreren Portionen aufgeteilt und jeweils an einer Source 15RPC-Säule mit einem Acetonitril/TFA-Gradienten wie oben beschrieben chromatographiert. Die Läufe werden nacheinander durchgeführt und die Fraktionen im gleichen Röhrchen gesammelt. Die resultierenden Chromatogramme sind deckungsgleich.

Das Fragment 4-11 (Rt. ca. 15 min) wird zusammengegeben, mit Wasser 1:1 verdünnt und dann lyophilisiert.

Die Ausbeute an Fragment 4-11 beträgt nach der Lyophilisation 1.1 g (1095 µmol) Bei einem Einsatz von 1150 µmol an spaltbarem Material ist die Ausbeute an Fragment 4-11 (95% d. Th.).

### Beispiel 13

### Präparative Chymotrypsinspaltung vom Gemisch Dihydro- / Octahydro-Lysobactin Substratkonzentration 3 mg/ml

2 x 0.995 g eines Gemisches aus Dihydro- (52%) und Octahydro-Lysobactin (37%) werden in je 33 ml Methanol gelöst und dann mit je 257 ml Spaltpuffer (0.1M Ammoniumhydrogencarbonat / 0.5M Harnstoff pH 8) versetzt. Vor der Enzymzugabe wird die Lösung im Trockenschrank auf 37°C erwärmt. 39.6 mg Chymotrypsin (39.6 ml Chymotrypsinlösung Wasser / Ethylenglykol 1:1, 1 mg/ml; 1:25; 37°C vorgewärmt) werden hinzugegeben und die Reaktion bei 37°C durchgeführt. Es werden 200 µl Aliquots nach 0.5, 1 Std. entnommen und die Enzymspaltung mit 200 µl 30% Acetonitril /0.1% TFA abgestoppt. Die Proben werden mit der HPLC parallel zur Enzymspaltung innerhalb von 15 min. analysiert (Retentionszeit Fragment 4-11 ca. 3.6 min, Fragment 1-3 (LLF) ca. 9.6 min., Fragment 1-3 (LL(3-Cyclohexyl)A) ca. 11.3 min) (Solvent A 0.1% TFA, Solvent B 60% Acetonitril/0.1% TFA, Gradient 0 min 30% B, 10 min 80% B, 11 min 100% B, 12 min 30% B, 15 min 30% B; Fluss: 0.7 ml/min, Temperatur: 40°C, UV-Detektion 210 nm). Die Enzymreaktionen werden nach 60 min. mit je 30 ml Acetonitril und ca. 2.5 ml TFA abgestoppt. Der pH-Wert der Lösung soll zwischen 1 und 2 liegen. Bis zur präparativen Trennung kann die Lösung bei -20°C gelagert werden.

### Beispiel 14

### Präparative Chymotrypsinspaltung vom Gemisch Dihydro- / Octahydro-Lysobactin Substratkonzentration 5 mg/ml

10 g Dihydro- (ca. 40%) und Octahydro-Lysobactin (ca. 60%) werden in 200 ml Methanol gelöst und dann mit 1700 ml Spaltpuffer (0.1 M Ammoniumhydrogencarbonat / 0.5 M Harnstoff pH 8) versetzt. Vor der Enzymzugabe wird die Lösung im Trockenschrank auf 37°C erwärmt. 400 mg Chymotrypsin (100 ml Chymotrypsinlösung Wasser / Ethylenglykol 1:1, 4 mg/ml; 1:25; 37°C vorgewärmt) werden hinzugegeben und die Reaktion bei 37°C durchgeführt. Es werden 200 µl Aliquots nach 0.5, 1 h entnommen und die Enzymspaltung mit 200 µl 30% Acetonitril / 0.1% TFA abgestoppt. Die Proben werden mit der HPLC parallel zur Enzymspaltung innerhalb von 15 min analysiert (Retentionszeit Fragment 4-11 ca. 3.6 min, Fragment 1-3 (LLF) ca. 9.6 min, Fragment 1-3 (LLA(3-cyclohexyl)) ca. 11.3 min) (Solvent A 0.1% TFA, Solvent B 60% Acetonitril/0.1% TFA, Gradient 0 min 30% B, 10 min 80% B, 11 min 100% B, 12 min 30% B, 15 min 30% B; Fluss: 0.7 ml/min, Temperatur: 40°C, UV-Detektion 210 nm). Die Enzymreaktion wird nach 60 min mit 75 ml Acetonitril und ca. 15 ml TFA abgestoppt. Der pH-Wert der Lösung soll zwischen 1 und 2 liegen. Bis zur präparativen Trennung kann die Lösung bei -20°C gelagert werden.

Das Fragment 4-11 wird wie oben beschrieben in mehreren Läufen durch präparative HPLC isoliert.

Die Aktivität der eingesetzten Chymotrypsincharge (70U/mg) wird durch eine Kontrollspaltung mit dem Protein Interleukin-4-Doppelmutein Arg(121) → Asp(121) / Tyr(124) → Asp(124) (BAYER Healthcare AG, D-Wuppertal) überprüft.

### Beispiel 15

### Subtilisin-Spaltung von Dihydro-Lysobactin

200 µg Dihydro-Lysobactin werden in 10 µl Methanol gelöst und dann mit 190 µl Spaltpuffer (0.1 M Ammoniumhydrogencarbonat / 0.5 M Harnstoff pH 8) versetzt. 4 µg Subtilisin (1:50) werden hinzugegeben und die Reaktion bei 37°C durchgeführt. Es werden 30 µl Aliquots nach 0, 0.5, 1, 3, 6 und 24 h entnommen und die Enzymspaltung mit 30 µl Acetonitril / 1% TFA abgestoppt. Die Proben werden bis zur Analyse bei -20°C gelagert.

Peptidsequenzen der Subtilisin-Spaltprodukte siehe Tabelle 3.

### Beispiel 16

### Subtilisin-Spaltung von Octahydro-Lysobactin

200 µg Octahydro-Lysobactin werden in 10 µl Methanol gelöst und dann mit 190 µl Spaltpuffer (0.1 M Ammoniumhydrogencarbonat / 0.5 M Harnstoff pH 8) versetzt. 4 µg Subtilisin (1:50) werden hinzugegeben und die Reaktion bei 37°C durchgeführt. Es werden 30 µl Aliquots nach 0, 0.5, 1, 3, 6 und 24 h entnommen und die Enzymspaltung mit 30 µl Acetonitril / 1% TFA abgestoppt. Die Proben werden bis zur Analyse bei -20°C gelagert.

Peptidsequenzen der Subtilisin-Spaltprodukte siehe Tabelle 3.

Die Aktivität der eingesetzten Subtilisincharge (ca.12 U/mg) wird durch eine Kontrollspaltung mit dem Protein Interleukin-4-Doppelmutein Arg(121) → Asp(121) / Tyr(124) → Asp(124) (BAYER Healthcare AG, D-Wuppertal) überprüft.

### Beispiel 17

### Thermolysin-Spaltung von Dihydro-Lysobactin

200 µg Dihydro-Lysobactin werden in 10 µl Methanol gelöst und dann mit 190 µl Spaltpuffer (0.1 M Tris-(hydroxymethyl)-aminomethan / 5 mM Calciumchlorid pH 7.45) versetzt. 4 µg Thermolysin (1:50) werden hinzugegeben und die Reaktion bei 37°C durchgeführt. Es werden 30 µl Aliquots nach 0, 0.5, 1, 3, 6 und 24 h entnommen und die Enzymspaltung mit 30 µl Acetonitril / 1% TFA abgestoppt. Die Proben werden bis zur Analyse bei -20°C gelagert.

Peptidsequenzen der Thermolysin-Spaltprodukte siehe Tabelle 4.

### Beispiel 18

### Thermolysin-Spaltung von Octahydro-Lysobactin

200 µg Octahydro-Lysobactin werden in 10 µl Methanol gelöst und dann mit 190 µl Spaltpuffer (0.1 M Ammoniumhydrogencarbonat / 0.5 M Harnstoff pH 8) versetzt. 4 µg Thermolysin (1:50) werden hinzugegeben und die Reaktion bei 37°C durchgeführt. Es werden 30 µl Aliquots nach 0, 0.5, 1, 3, 6 und 24 h entnommen und die Enzymspaltung mit 30 µl Acetonitril / 1% TFA abgestoppt. Die Proben werden bis zur Analyse bei -20°C gelagert.

Peptidsequenzen der Thermolysin-Spaltprodukte siehe Tabelle 4.

Die Aktivität der eingesetzten Thermolysincharge (ca. 55U/mg) wird durch eine Kontrollspaltung mit dem Protein Interleukin-4-Doppelmutein Arg(121) → Asp(121) / Tyr(124) → Asp(124) (BAYER Healthcare AG, D-Wuppertal) überprüft.

### Beispiel 19

### Papain-Spaltung von Dihydro-Lysobactin

200 µg Dihydro-Lysobactin werden in 10 µl Methanol gelöst und dann mit 190 µl Spaltpuffer (0.1 M Natriumphosphat / 10 mM Cystein, 2 mM EDTA pH 6.5) versetzt. 4 µg Papain (1:50) werden hinzugegeben und die Reaktion bei 37°C durchgeführt. Es werden 30 µl Aliquots nach 0, 0.5, 1, 3, 6 und 24 h entnommen und die Enzymspaltung mit 30 µl Acetonitril / 1% TFA abgestoppt. Die Proben werden bis zur Analyse bei -20°C gelagert.

Peptidsequenzen der Papain-Spaltprodukte siehe Tabelle 5.

### Beispiel 20

### Papain-Spaltung von Octahydro-Lysobactin

200 µg Octahydro-Lysobactin werden in 10 µl Methanol gelöst und dann mit 190 µl Spaltpuffer (0.1 M Natriumphosphat / 10 mM Cystein, 2 mM EDTA pH 6.5) versetzt. 4 µg Papain (1:50) werden hinzugegeben und die Reaktion bei 37°C durchgeführt. Es werden 30 µl Aliquots nach 0, 0.5, 1, 3, 6 und 24 h entnommen und die Enzymspaltung mit 30 µl Acetonitril / 1% TFA abgestoppt. Die Proben werden bis zur Analyse bei -20°C gelagert.

Peptidsequenzen der Papain-Spaltprodukte siehe Tabelle 5.

Die Aktivität der eingesetzten Papaincharge (ca.11U/mg) wird durch eine Kontrollspaltung mit dem Protein Interleukin-4-Doppelmutein Arg(121) → Asp(121) / Tyr(124) → Asp(124) (BAYER Healthcare AG, D-Wuppertal) überprüft.

### Beispiel 21

### Proteinase K-Spaltung von Dihydro-Lysobactin

200 µg Dihydro-Lysobactin werden in 10 µl Methanol gelöst und dann mit 190 µl Spaltpuffer (0.1 M Natriumtetraborat pH 9) versetzt. 4 µg Proteinase K (1:50) werden hinzugegeben und die Reaktion bei 37°C durchgeführt. Es werden 30 µl Aliquots nach 0, 0.5, 1, 3, 6 und 24 h entnommen und die Enzymspaltung mit 30 µl Acetonitril / 1 % TFA abgestoppt. Die Proben werden bis zur Analyse bei -20°C gelagert.

Peptidsequenzen der Proteinase K-Spaltprodukte siehe Tabelle 6.

### Beispiel 22

### Proteinase K-Spaltung von Octahydro-Lysobactin

200 µg Octahydro-Lysobactin werden in 10 µl Methanol gelöst und dann mit 190 µl Spaltpuffer (0.1 M Natriumtetraborat pH 9) versetzt. 4 µg Proteinase K (1:50) werden hinzugegeben und die Reaktion bei 37°C durchgeführt. Es werden 30 µl Aliquots nach 0, 0.5, 1, 3, 6 und 24 h entnommen und die Enzymspaltung mit 30 µl Acetonitril / 1% TFA abgestoppt. Die Proben werden bis zur Analyse bei -20°C gelagert.

Peptidsequenzen der Proteinase K-Spaltprodukte siehe Tabelle 6.

Die Aktivität der eingesetzten Proteinase K Charge (ca.30U/mg) wird durch eine Kontrollspaltung mit dem Protein Interleukin-4-Doppelmutein Arg(121) → Asp(121) / Tyr(124) → Asp(124) (BAYER Healthcare AG, D-Wuppertal) überprüft.

### Beispiel 23

### Bromelain-Spaltung von Dihydro-Lysobactin

200 µg Dihydro-Lysobactin werden in 10 µl Methanol gelöst und dann mit 190 µl Spaltpuffer (0.1 M Natriumphosphat, 10 mM Cystein, 2 mM EDTA pH 6.5) versetzt. 4 g Bromelain (1:50) werden hinzugegeben und die Reaktion bei 37°C durchgeführt. Es werden 30 µl Aliquots nach 0, 0.5, 1, 3, 6 und 24 h entnommen und die Enzymspaltung mit 30 µl Acetonitril / 1% TFA abgestoppt. Die Proben werden bis zur Analyse bei -20°C gelagert.

Peptidsequenzen der Bromelain-Spaltprodukte siehe Tabelle 7.

### Beispiel 24

### Bromelain-Spaltung von Octahydro-Lysobactin

200 µg Octahydro-Lysobactin werden in 10 µl Methanol gelöst und dann mit 190 µl Spaltpuffer (0.1 M Natriumphosphat, 10 mM Cystein, 2 mM EDTA pH 6.5) versetzt. 4 µg Bromelain (1:50) werden hinzugegeben und die Reaktion bei 37°C durchgeführt. Es werden 30 µl Aliquots nach 0, 0.5, 1, 3, 6 und 24 h entnommen und die Enzymspaltung mit 30 µl Acetonitril / 1% TFA abgestoppt. Die Proben werden bis zur Analyse bei -20°C gelagert.

Peptidsequenzen der Bromelain-Spaltprodukte siehe Tabelle 7.

Die Aktivität der eingesetzten Bromelain Charge (ca.4U/mg) wird durch eine Kontrollspaltung mit dem Protein Interleukin-4-Doppelmutein Arg(121) → Asp(121) / Tyr(124) → Asp(124) (BAYER Healthcare AG, D-Wuppertal) überprüft.

### Beispiel 25

### Enzymatische Synthese von Dihydro-Lysobactin mit Chymotrypsin

800 µg Peptid Leu-Leu-PheOMe und 100 µg Peptid 4-11 werden in 200 µl Methanol gelöst und dann mit 200 µl Synthesepuffer (0.1 M Natriumtetraborat pH 9) versetzt. 24 µg Chymotrypsin werden hinzugegeben und die Reaktion bei 37°C durchgeführt. Es werden 30 µl Aliquots nach 0, 0.5, 1, 3, 6 und 24 h entnommen und die Synthese mit 30 µl Acetonitril / 1% TFA abgestoppt. Die Proben werden bis zur Analyse bei -20°C gelagert.

Der Nachweis von Dihydro-Lysobactin erfolgt mittels HPLC und CZE.

### Beispiel 26

### Enzymatische Synthese von Dihydro-Lysobactinderivaten mit Chymotrypsin

800 µg Peptid Boc-Leu-Leu-PheOMe werden in 200 µl Tetrachlormethan gelöst und dann mit 200 µl Synthesepuffer (0.1 M Natriumtetraborat pH 9) der 100 µg Peptid 4-11 enthält versetzt. 24 µg Chymotrypsin werden hinzugegeben und die Reaktion bei 37°C durchgeführt. Es werden 30 µl Aliquots nach 0, 0.5, 1, 3, 6 und 24 h entnommen und die Synthese mit 30 µl Acetonitril / 1% TFA abgestoppt. Die Proben werden bis zur Analyse bei -20°C gelagert.

Der Nachweis von Dihydro-Lysobactinderivaten erfolgt mittels HPLC und CZE.

### Beispiel 27

### Enzymatische Synthese von Octahydro-Lysobactin mit Chymotrypsin

800 µg Peptid Leu-Leu-Ala(3-cyclohexyl)OMe und 100 µg Peptid 4-11 werden in 200 µl Methanol gelöst und dann mit 200 µl Synthesepuffer (0.1 M Natriumtetraborat pH 9) versetzt. 24 µg Chymotrypsin werden hinzugegeben und die Reaktion bei 37°C durchgeführt. Es werden 30 µl Aliquots nach 0, 0.5, 1, 3, 6 und 24 h entnommen und die Synthese mit 30 µl Acetonitril / 1% TFA abgestoppt. Die Proben werden bis zur Analyse bei -20°C gelagert.

Der Nachweis von Octahydro-Lysobactin erfolgt mittels HPLC und CZE.

### Beispiel 28

### N-terminale Sequenzanalyse

3 nmol in 60% Acetonitril/0.1% TFA gelöste Fragmente werden auf ein Sequenzer-sheet, das mit Polybren^{R} vorinkubiert ist, geladen. Die Proteine werden mit dem normalen Sequenzerzyklus sequenziert. Die PTH-Aminosäuren werden mittels Online-HPLC mit Hilfe eines 40 pmol PTH-Standards identifiziert. Die nicht proteinogenen Aminosäuren werden durch ihre relative Lage zu den Standardaminosäuren identifiziert. Die Reinheit der Peptide wird über die Aminosäure des 1.PTH-Zyklus abgeschätzt. Die verschiedenen Peptide werden über 4 bis 12 Stufen sequenziert. Die Tabellen 1 bis 7 zeigen die bestimmten Proteinsequenzen.

**Tabelle 1: Peptidsequenzen der Substrate**

| Peptide | Bestimmte Peptidsequenzen der Substrate |
|---|---|
| 1. Dihydro-Lysobactin | Leu-Leu-Phe-Leu(OH)-Leu-Arg-Ile-(allo)Thr-Gly-Asn(OH)-Ser |
| 2. Octahydro-Lysobactin | Leu-Leu-PTHAla(3-cyclohexyl)*-Leu(OH)-Leu-Arg-Ile-(allo)Thr-Gly-Asn(OH)-Ser |

**Tabelle 2: Sequenzanalyse von verschiedenen Peptiden (1-3) bzw. Peptidfragmenten (1-3) der Chymotrypsin-Spaltung.**

| Peptide | Bestimmte Peptidsequenzen der Chymotrypsin-Spaltprodukte |
|---|---|
| 1. Peptid 4-11 | Leu(OH)-Leu-Arg-Ile-(allo)Thr-Gly-Asn(OH)-Ser |
| 2. Peptid 1-3 | Leu-Leu-Phe |
| 3. Peptid 1-3 | Leu-Leu-PTHAla(3-cyclohexyl)* |

| | |
|---|---|
| *Die PTHAla(3-cyclohexyl) ist mit dem verwendeten PTH-System als Peak nicht detektierbar. | |

**Tabelle 3: Sequenzanalyse von verschiedenen Peptiden bzw. Peptidfragmenten der Subtilisin-Spaltung von Dihydro- und Octahydro-Lysobactin (1-4). Das Spaltprodukt 4-10 entsteht in größerem Umfang erst nach 24 h.**

| Peptide | Bestimmte Peptidsequenzen der Subtilisin-Spaltprodukte |
|---|---|
| 1. Peptid 4-11 | Leu(OH)-Leu-Arg-Ile-(allo)Thr-Gly-Asn(OH)-Ser |
| 2. Peptid 4-10 | Leu(OH)-Leu-Arg-Ile-(allo)Thr-Gly-Asn(OH)-Ser |
| 3. Peptid 1-3 | Leu-Leu-Phe |
| 4. Peptid 1-3 | Leu-Leu-PTHAla(3-cyclohexyl)* |

| | |
|---|---|
| *Die PTHAla(3-cyclohexyl) ist mit dem verwendeten PTH-System als Peak nicht detektierbar. | |

**Tabelle 4: Sequenzanalyse von verschiedenen Peptiden bzw. Peptidfragmenten der Thermolysin-Spaltung von Dihydro- und Octahydro-Lysobactin (1-3).**

| Peptide | Bestimmte Peptidsequenzen der Thermolysin-Spaltprodukte |
|---|---|
| 1. Peptid 3-11 | Phe-Leu(OH)-Leu-Arg-Ile-(allo)Thr-Gly-Asn(OH)-Ser |
| 2. Peptid 3-11 | PTHAla(3-cyclohexyl)*-Leu(OH)-Leu-Arg-Ile-(allo)Thr-Gly-Asn(OH) |
| 3. Peptid 1-2 | Leu-Leu |

| | |
|---|---|
| *Die PTHAla(3-cyclohexyl) ist mit dem verwendeten PTH-System als Peak nicht detektierbar. | |

**Tabelle 5: Sequenzanalyse von verschiedenen Peptiden bzw. Peptidfragmenten der Papain-Spaltung von Dihydro- (1, 2, 3, 4) und Octahydro-Lysobactin (1, 2, 3, 5). Das Spaltprodukt 4-10 entsteht in größerem Umfang erst nach 24 h.**

| Peptide | Bestimmte Peptidsequenzen der Papain-Spaltprodukte |
|---|---|
| 1. Peptid 5-11 | Leu-Arg-Ile-(allo)Thr-Gly-Asn(OH)-Ser |
| 2. Peptid 5-9 | Leu-Arg-Ile-(allo)Thr-Gly |
| 3. Peptid 10-11 | Asn(OH)-Ser |
| 4. Peptid 1-4 | Leu-Leu-Phe-Leu(OH) |
| 5. Peptid 1-4 | Leu-Leu-PTHAla(3-cyclohexyl)*-Leu(OH) |

| | |
|---|---|
| *Die PTHAla(3-cyclohexyl) ist mit dem verwendeten PTH-System als Peak nicht detektierbar. | |

**Tabelle 6: Sequenzanalyse von verschiedenen Peptiden bzw. Peptidfragmenten der Proteinase K-Spaltung von Dihydro- (1, 2) und Octahydro-Lysobactin (3, 4).**

| Peptide | Bestimmte Peptidsequenzen der Proteinase K-Spaltprodukte |
|---|---|
| 1. Peptid 4-11 | Leu(OH)-Leu-Arg-Ile-(allo)Thr-Gly-Asn(OH)-Ser |
| 2. Peptid 5-11 | Leu-Arg-Ile-(allo)Thr-Gly-Asn(OH)-Ser |
| 4. Peptid 4-10 | Leu(OH)-Leu-Arg-Ile-(allo)Thr-Gly-Asn(OH) |
| 5. Peptid 5-10 | Leu-Arg-Ile-(allo)Thr-Gly-Asn(OH) |
| 6. Peptid 1-2 | Leu-Leu |

**Tabelle 7: Sequenzanalyse von verschiedenen Peptiden bzw. Peptidfragmenten der Bromelain-Spaltung von open-chain-Lysobactin (1, 4), Dihydro- (2, 4) und Octahydro-Lysobactin (3, 4).**

| Peptide | Bestimmte Peptidsequenzen der Bromelain-Spaltprodukte |
|---|---|
| 2. Peptid 1- 9 | Leu-Leu-Phe-Leu(OH)-Leu-Arg-Ile-(allo)Thr-Gly |
| 3. Peptid 1- 9 | Leu-Leu-PTHAla(3-cyclohexyl)*-Leu(OH)-Leu-Arg-Ile-(allo)Thr-Gly |
| 4. Peptid 10-11 | Asn(OH)-Ser |

| | |
|---|---|
| *Die PTHAla(3-cyclohexyl) ist mit dem verwendeten PTH-System als Peak nicht detektierbar. | |

### Beispiel 29

### Aminosäureanalyse

Die Aminosäureanalyse stellt einen wichtigen qualitativen und quantitativen Parameter für die Charakterisierung von Proteinen dar. Neben dem Proteingehalt wird bei bekannter Primärstruktur die Anzahl der Einzelaminosäuren ermittelt. Die Aminosäureanalyse Lysobactinderivate bzw. Peptidfragmente ist in guter Übereinstimmung mit den theoretischen Werten aus der Primärstruktur (Tabelle 8). Nichtproteinogene Aminosäuren werden nur bei dem Vorhandensein von entsprechenden Standards quantifiziert.

100 µg der Lysobactinderivate bzw. Peptidfragmente werden in 200 µl 6 N Salzsäure gelöst und 1 h bei 166°C hydrolysiert. Ca. 5 nmol der Proben wird auf den Aminosäureanalysator gegeben. Die Menge an Aminosäure wird über einen 4 nmol Aminosäurestandard ermittelt.

**Tabelle 8: Aminosäureanalyse von Dihydro-, Octahydro-Lysobactin, Dihydro- + Octahydro- Lysobactin, Fragment 4-11 und 1-3. Die ganzen Zahlen sind auf Ile = 1 bzw. Leu = 2 bezogen.**

| **Aminosäure** | **Peptid 1-3** | **theoret. Zahlen** | **Peptid 4-11** | **theoret. Zahlen** | **Dihydro- + Octahydro-Lysobactin** | **Dihydro-Lysobactin** | **Octahydro-Lysobactin** | **theoret. Zahlen** |
|---|---|---|---|---|---|---|---|---|
| **Asx(OH)** | | | **n.d.** | **1** | **n.d.** | **n.d.** | **n.d.** | **1** |
| **Asx** | | | | | | | | |
| **alloTHR** | | | **1.04** | **1** | **0.91** | **1.11** | **1.01** | **1** |
| **Ser** | | | **0.59** | **1** | **0.89** | **0.99** | **0.90** | **1** |
| **Glx** | | | | | | | | |
| **Gly** | | | **1.11** | **1** | **1.15** | **1.17** | **1.12** | **1** |
| **Ala** | | | | | | | | |
| **Val** | | | | | | | | |
| **Met** | | | | | | | | |
| **Ile*** | | | **1.00** | **1** | **1.00** | **1.00** | **1.00** | **1** |
| **Leu*** | **2.00** | **2** | **1.04** | **1** | **2.93** | **2.33** | **2.53** | **3** |
| **Tyr** | | | | | | | | |
| **Phe** | **1.01** | **1** | | | **0.55** | **1.08** | | **1** |
| **Ala(3-cyclohexyl)** | | | | | **n.d.** | | **n.d.** | **1** |
| **Leu(OH)** | | | **n.d.** | **1** | **n.d.** | **n.d.** | **n.d.** | **1** |
| **Lys** | | | | | | | | |
| **Arg** | | | **1.06** | **1** | **1.05** | **1.18** | **1.15** | **1** |
| **Phe(OH)** | | | | | | | | **1** |
| | | | | | | | | |
| **Summe AS** | **3.01** | **3** | **5.83** | **8** | **9.39** | **8.85** | **7.71** | |

### Beispiel 30

### Reverse Phase Chromatographie

Bei der HPLC-Chromatographie von Proteinen an chemisch gebundene Umkehrphasen kommt es über eine hydrophoben Wechselwirkung der Proteine zu einer Bindung an die verwendete Phase. Die Peptide werden gemäß der Stärke ihrer Bindung an die stationäre Phase von organischen Lösungsmitteln (mobile Phase) verdrängt. Aus diesem Grund ist diese Methode ein gutes Kriterium für die Beurteilung der Reinheit eines Peptids und zur Kontrolle der Geschwindigkeit der enzymatischen Spaltung und der entstehenden Spaltprodukte. Die Peptide Dihydro-Lysobactin und Octahydro-Lysobactin eluieren bei ca. 35 min und ca. 38 min, das Fragment 4-11 bei ca. 16 min, 1-3 (LLF) bei ca. 31 min und 1-3 (LLA(3-cyclohexyl)) bei ca. 37 min von der RP-18-Phase. Die Abbildung 1 zeigt den zeitlichen Verlauf einer präparativen enzymatischen Spaltung mit Chymotrypsin (Beispiel 11).

Ca. 20 µg der enzymatischen Spaltprodukte bzw. der Ausgangsverbindungen Dihydro-Lysobactin und Octahydro-Lysobactin oder des Gemisch werden an einer Zobax 300SB-C₁₈-column (4.6 mm x 150 mm; 3.5 µm Material; 300 Angström Porendurchmesser) chromatographiert. Als Eluent wird ein Acetonitril/TFA-Gradient verwendet. Bedingungen: Solvent A 0.1% TFA, Solvent B 60% Acetonitril/0.1% TFA; Fluss 0.7 ml/min, Säulentemperatur 40°C, UV-Detektion 210 nm, Lösungsmittel A 0.1 % TFA, Lösungsmittel B 0.1 % TFA / 60% Acetonitril; Gradient: 0 min 0% B, 2 min 10% B, 50 min 80% B, 52 min 100% B, 55 min 0% B, 60 min 0% B.

### Beispiel 31

### Kapillarzonenelektrophorese (CZE)

Die Kapillarelektrophorese erlaubt die Trennung von Peptiden und Proteinen aufgrund ihrer Ladung im elektrischen Feld. Die Güte der Trennung hängt dabei vom Puffer, dem pH-Wert, der Temperatur und den verwendeten Additiven ab. Als Kapillaren werden sogenannte "fused silica" Säulen mit einem Innendurchmesser von 50-100 µm eingesetzt. Diese Methode ist ein sehr gutes Kriterium für die Beurteilung der Reinheit eines Peptids und zur Kontrolle der Entstehung von enzymatischen Spaltprodukten. Die Peptide Dihydro-Lysobactin und Octahydro-Lysobactin eluieren bei ca. 21 min, das Fragment 4-11 bei ca. 18 min, 1-3 (LLF) bei ca. 24 min, 1-3 (LLA(3-cyclohexyl)) bei ca. 22 min, die deamidierten Formen als Doppelpeak bei ca. 30 min (1-11) und 24 min (4-11) von der Kapillarsäule. Die Abbildung 2 zeigt den zeitlichen Verlauf einer enzymatischen Spaltung von Octahydro-Lysobactin mit Chymotrypsin (Beispiel 5). Man sieht deutlich die starke Zunahme der deamidierten Produkte nach 24 h im Puffer.

Ca. 4 ng der enzymatischen Spaltprodukte bzw. der Ausgangsverbindungen Dihydro-Lysobactin und Octahydro-Lysobactin oder des Gemisch werden mittels Kapillarelektrophorese an einer Glassäule (Länge 72 cm, Innendurchmesser 50 µm) untersucht. Bedingungen: Stromstärke 90 µA, Säulentemperatur 25°C, 100 mM Phosphatpuffer pH 3.0, UV-Detektion 210 nm, Aufgabe unter Druck 3 Sekunden.

### Beispiel 32

### Molekulargewichtsbestimmung durch HPLC-ESI-MS

Peptide und enzymatische Spaltprodukte werden mittels RP-18-HPLC-Chromatographie getrennt und das Molekulargewicht durch Elektronenspray-Ionisation (ESI) bestimmt.

Ca. 100 µg Chymotrypsinspaltung vom Gemisch aus Dihydro-Lysobactin und Octahydro-Lysobactin werden mit einer Zorbax C18-HPLC-Säule unter folgenden Bedingungen aufgetrennt: Solvent A 0.1% TFA, Solvent B 60% Acetonitril/0.1% TFA; Fluss 0.7 ml/min, Säulentemperatur 40°C, UV-Detektion 210 nm, Lösungsmittel A 0.1% TFA, Lösungsmittel B 0.1% TFA / 60% Acetonitril; Gradient: 0 min 0% B, 2 min 10% B, 50 min 80% B, 52 min 100% B, 55 min 0% B, 60 min 0% B. Die Peptide werden in die Atmosphärendruckionenquelle des Massenspektrometers überführt und dort ionisiert. Von dort werden die Ionen in den Hochvakuumbereich des Massenspektrometers überführt und detektiert. Die Tabelle 9 zeigt die bestimmten Molekulargewichte.

**Tabelle 9: Molekulargewichte von Dihydro-Lysobactin, Octahydro-Lysobactin und von enzymatischen Spaltprodukten im Vergleich mit den theoretischen Molekulargewichten (MW) in Dalton.**

| **Peptide** | **MW in Da** | **Theoretische MW in Da** |
|---|---|---|
| 1. Dihydro-Lysobactin | 1279 | 1278.5 |
| 2. Octahydro-Lysobactin | 1285 | 1284.6 |
| 3. Peptid 4-11 | 905 | 905 |
| 4. Peptid 1-3 (LLF) | 391 | 391 |
| 5. Peptid 1-3 (LLA(3-cyclohexyl)) | 397 | 397 |
| 6. Peptid 1-9 | 1062 | 1061.5 |
| 7. Peptid 1-9 (A(3-cyclohexyl)) | 1068 | 1067.6 |

### Beispiel 33

### Präparative Chymotrypsinspaltung vom Gemisch Dihydro- / Oktahydro-Lysobactin

18.27 g Dihydro- und Okta-Lysobactin (ca. 5:4) werden in 365 ml Methanol gelöst und mit Chymotrypsin (731 mg) und Spaltpuffer auf 3654 ml verdünnt. Die Reaktion wird 30 min. bei 37°C durchgeführt und dann mit 20 ml TFA und 150 ml Acetonitril abgestoppt. Vor der Enzymzugabe werden die Lösungen im Trockenschrank auf 37°C erwärmt. Es werden 200 µl Aliquots nach 0 und 0.5 h entnommen und die Enzymspaltung mit 200 µl 0.1% TFA in 30% Acetonitril / 70% Wasser abgestoppt. Die Proben werden mit der HPLC analysiert (Retentionszeit Fragment 4-11 ca. 3.6 min., Fragment 1-3 (LLF) ca. 9.6 min., Fragment 1-3 (LL(Hexahydro)F) ca. 11.3 min.) (Eluent A: 0.1% TFA in Wasser, Eluent B: 0.1% TFA in 60% Acetonitril/ 40% Wasser, Gradient: 0 min 30% B, 10 min 80% B, 11 min 100% B, 12 min 30% B, 15 min 30% B; Fluss: 0.7 ml/min, Säulentemperatur: 40°C, Detektion: 210 nm). Alternativ wird Methode 11 verwendet. Die Lösung wird auf 9 x 500 ml Portionen verteilt und bis zur präparativen RP-Trennung bei -70°C tiefgefroren. Das Fragment 4-11 wird in mehreren Läufen durch präparative HPLC isoliert.

### Präparative Trennung der Fragmente 1-3 und 4 -11:

Ca. 800 ml der Spaltlösung werden über eine Kartusche (0.2 µm) filtriert und in zwei Portionen zu ca. 400 ml an einer Source 15RPC-Säule (Säulengröße: 2360 ml) mit einem Methanol/TFA-Gradienten chromatographiert. Eluent A: 0.1% TFA in Wasser, Eluent B: 0.1% TFA in 100% Methanol; Fluss: 30 ml/min.; Detektion 215 nm. Der Gradient wird nach Säulenvolumen gefahren: Nach dem Auftrag wird mit 3.6 Säulenvolumen Eluent A gewaschen, dann in 18 Säulenvolumen nach 45%B, in 0.67 Säulenvolumen nach 100% B, 1.3 Säulenvolumen 100%B, in 0.67 nach 0%B, 7 Säulenvolumen Eluent A zur Äquilibrierung.

Als Produkt erhält man 10.36 g (77% d. Th.) Fragment 4-11.

HPLC/UV-Vis (Methode 4): Rₜ = 0.5 min.

LC-MS (Methode 1): Rₜ = 1.0 min;

MS (ESIpos.): *m*/*z* (%) = 453.6 (100) [M + 2H]²⁺, 906 (10) [M + H]⁺.

MS (ESIneg.): *m*/*z* (%) = 904 (100) [M - H]⁻.

## Patentansprüche

1. Verwendung von Lysobactinfragment 4-11 zur Synthese von Lysobactin-Derivaten.

2. Verfahren zur Herstellung von open-chain-Lysobactin-Derivaten, **dadurch gekennzeichnet, dass** das Lysobactinfragment 4-11 mit einem Tripeptid mit einer C-terminalen aromatischen oder hydrophoben Aminosäure in einem Puffermedium unter Zugabe eines C₁₋₄-Alkohols umgesetzt wird, wobei das Tripeptid in Form der freien Säure oder eines Esters vorliegt und wobei die Konzentration des C₁₋₄-Alkohols im Reaktionsmedium größer als 40% ist.

3. Verfahren nach Anspruch 2, **dadurch gekennzeichnet, dass** als C₁₋₄-Alkohol Methanol eingesetzt wird.
